# EUROPEAN PATENT APPLICATION

(11) **EP 4 595 963 A1**
(43) Date of publication of application: **06.08.2025**
(21) Application number: 23870858.0
(22) Date of filing: 26.09.2023
(51) Int. Cl.: A61K 31/444, A61K 31/4439, A61K 31/506, A61K 31/4995, A61K 31/4545, A61K 39/395, A61P 35/00

(54) **PHARMACEUTICAL COMBINATION OF FAK INHIBITOR AND SUBSTANCE FOR INDUCING IMMUNOGENIC CELL DEATH AND USE**

(30) Priority: 30.09.2022 CN 202211211068; 19.09.2023 CN 202311207394
(71) Applicant: Inxmed (Nanjing) Co., Ltd., Nanjing, Jiangsu 210061 (CN)
(72) Inventor: ZHANG, Baoyuan, Nanjing, Jiangsu 210061 (CN); LIU, Xuebin, Nanjing, Jiangsu 210061 (CN); ZHANG, Ping, Nanjing, Jiangsu 210061 (CN); PANG, Ran, Nanjing, Jiangsu 210061 (CN); WANG, Zaiqi, Nanjing, Jiangsu 210061 (CN)
(74) Representative: D Young & Co LLP
(86) International application number: PCT/CN2023/121715
(87) International publication number: WO 2024/067631

(57) **Abstract**

Use of a composition of an FAK inhibitor, a substance for inducing immunogenic cell death, and an immune checkpoint inhibitor in preparing a drug for treating a tumor. The substance for inducing immunogenic cell death is an RNA polymerase II inhibitor, an ALK/ROS1 inhibitor, a KRAS G12C inhibitor, or a KRAS G12D inhibitor. The immune checkpoint inhibitor is an anti-PD-1/PD-L1 antibody, a PD-1/PD-L1 small molecule inhibitor, or a TIGIT antibody. Provided is a pharmaceutical combination product of an FAK inhibitor, a substance for inducing immunogenic cell death, and an immune checkpoint inhibitor for use in treating a tumor. Provided is an FAK inhibitor for use in enhancing immunogenic cell death induced by the substance for inducing immunogenic cell death in the treatment of a tumor.

## Description

This application claims the priority of Chinese Patent Application No. 202211211068.5 filed on September 30, 2022 and Chinese Patent Application No. 202311207394.3 filed on September 19, 2023. The disclosures of the above-mentioned Chinese patent applications are cited in their entirety as part of this application.

### TECHNICAL FIELD

The present disclosure belongs to the field of medicinal chemistry. Specifically, the present disclosure relates to the treatment of tumors using a focal adhesion kinase (FAK) inhibitor in combination with other drugs.

### BACKGROUND

Tumor is the second largest killer threatening people's health. Immunogenic cell death (ICD) is based on the superposition effect of programmed cell death. An intracellular pressure signal may be activated when cancer cells are exposed to chemotherapy or targeted medicaments. This kind of pressure signal includes endoplasmic reticulum pressure (ER stress) and reactive oxygen species pressure (oxidative stress). Under the action of the pressure signal, cells will try to repair the pressure first. The cells will start the programmed death process, if the damage caused by the pressure exceeds the repair ability of the cells. This process is often accompanied by the release of a class of damage associated molecular patterns (DAMPs). This kind of DAMPs will be specifically recognized by pattern recognition receptors on antigen-presenting cells (APC) in the body, which will induce the maturation, differentiation and activation of APC, and then gradually present it to immune cells such as effector T cells, thus making immune cells generate antigen memory. When tumor cells from the same source are found again, immune cells will specifically recognize and kill the tumor cells. The new tumor-specific immune response initiated by ICD can increase the sensitivity to an immune checkpoint inhibitor (ICI), so as to enhance the effect of the immune checkpoint inhibitor, and produce anti-tumor response with lasting immune memory.

FAK, also known as protein tyrosine kinase 2 (PTK2), is a non-receptor tyrosine kinase and a key component of focal adhesion complex. FAK plays an important role in mediating integrin and growth factor signals to regulate the invasion, proliferation and survival of tumor cells.

At present, the effect of an immunogenic cell death inducer in clinical practice needs to be improved. Therefore, it is still necessary to find a way to improve the efficacy of a single immunogenic cell death inducer and to further overcome the problem of drug resistance.

### SUMMARY

One aspect of the present disclosure provides the use of an FAK inhibitor, an immunogenic cell death inducer and an immune checkpoint inhibitor in the manufacture of a medicament for treating tumors in subjects, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides a pharmaceutical combination product of an FAK inhibitor, an immunogenic cell death inducer, and an immune checkpoint inhibitor for treating tumors in a subject, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides a method for treating tumors, which comprises administering a therapeutically effective amount of an FAK inhibitor, an immunogenic cell death inducer and an immune checkpoint inhibitor to a subject in need, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides a kit or a pharmaceutically acceptable composition comprising: (a) an FAK inhibitor; (b) an immunogenic cell death inducer; and (c) an immune checkpoint inhibitor, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides the use of an FAK inhibitor and an immunogenic cell death inducer in the manufacture of a medicament for treating tumors, wherein the FAK inhibitor is used to enhance immunogenic cell death induced by the immunogenic cell death inducer, and the immunogenic cell death inducer is an RNA polymerase II inhibitor, an ALK/ROS1 inhibitor, a KRAS G12C inhibitor or a KRAS G12D inhibitor.

Yet another aspect of the present disclosure provides an FAK inhibitor for enhancing immunogenic cell death induced by an immunogenic cell death inducer in the treatment of tumors, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides a method for treating tumors, which comprises administering a therapeutically effective amount of an FAK inhibitor and an immunogenic cell death inducer to a subject in need, wherein the FAK inhibitor is used to enhance the immunogenic cell death induced by the immunogenic cell death inducer, and the immunogenic cell death inducer is an RNA polymerase II inhibitor, an ALK/ROS1 inhibitor, a KRAS G12C inhibitor or a KRAS G12D inhibitor.

Yet another aspect of the present disclosure provides the use of an FAK inhibitor, an immunogenic cell death inducer and an immune checkpoint inhibitor in the manufacture of a medicament for the combined treatment of tumors, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides the use of an FAK inhibitor in the manufacture of a combined medicament with an immunogenic cell death inducer and an immune checkpoint inhibitor for treating tumors, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides the use of an immunogenic cell death inducer in the manufacture of a combined medicament with an FAK inhibitor and an immune checkpoint inhibitor for treating tumors, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides the use of an immune checkpoint inhibitor in the manufacture of a combined medicament with an FAK inhibitor and an immunogenic cell death inducer for treating tumors, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides the use of an FAK inhibitor in the manufacture of a medicament for the combined treatment of tumors with an immunogenic cell death inducer and an immune checkpoint inhibitor, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides the use of an immunogenic cell death inducer in the manufacture of a medicament for the combined treatment of tumors with an FAK inhibitor and an immune checkpoint inhibitor, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides the use of an immune checkpoint inhibitor in the manufacture of a medicament for the combined treatment of tumors with an FAK inhibitor and an immunogenic cell death inducer, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides a kit comprising: an FAK inhibitor; and instructions, which indicate that the FAK inhibitor can be used for the combined treatment of tumors with an immunogenic cell death inducer and an immune checkpoint inhibitor, and wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides a kit comprising: an immunogenic cell death inducer; and instructions, which indicate that the immunogenic cell death inducer can be used for the combined treatment of tumors with an FAK inhibitor and an immune checkpoint inhibitor, and wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides a kit comprising: an immune checkpoint inhibitor; and instructions, which indicate that the immune checkpoint inhibitor can be used for the combined treatment of tumors with an FAK inhibitor and an immunogenic cell death inducer, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides a method for treating tumors, which comprises administering a therapeutically effective amount of an FAK inhibitor and an immunogenic cell death inducer to a subject in need, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides a pharmaceutical combination product of an FAK inhibitor and an immunogenic cell death inducer, which is used for treating tumors in a subject in need, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides the use of an FAK inhibitor and an immunogenic cell death inducer in the manufacture of a combined medicament for treating tumors, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides the use of an FAK inhibitor in the manufacture of a combined medicament with an immunogenic cell death inducer for treating tumors, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides the use of an immunogenic cell death inducer in the manufacture of a combined medicament with an FAK inhibitor for treating tumors, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides the use of an FAK inhibitor and an immunogenic cell death inducer in the manufacture of a medicament for the combined treatment of tumors, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides the use of an FAK inhibitor in the manufacture of a medicament for the combined treatment of tumors with an immunogenic cell death inducer, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides the use of an immunogenic cell death inducer in the manufacture of a medicament for the combined treatment of tumors with an FAK inhibitor, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides a kit comprising: an FAK inhibitor; and instructions, which indicate that the FAK inhibitor can be used for the combined treatment of tumors with an immunogenic cell death inducer, wherein the immunogenic cell death inducer is an RNA polymerase II inhibitor, an ALK/ROS1 inhibitor, a KRAS G12C inhibitor or a KRAS G12D inhibitor.

Yet another aspect of the present disclosure provides a kit comprising: an immunogenic cell death inducer; and instructions, which indicate that the immunogenic cell death inducer can be used for the combined treatment of tumors with an FAK inhibitor, wherein the immunogenic cell death inducer is an RNA polymerase II inhibitor, an ALK/ROS1 inhibitor, a KRAS G12C inhibitor or a KRAS G12D inhibitor.

Optionally, the FAK inhibitor is IN10018, Defactinib, GSK2256098, PF-00562271, VS-4718, APG-2449, AMP945, AMP886 or pharmaceutically acceptable salts thereof, alternatively the FAK inhibitor is IN10018, Defactinib, AMP945 or pharmaceutically acceptable salts thereof; and yet alternatively, the FAK inhibitor is IN10018 or a pharmaceutically acceptable salt thereof, alternatively IN10018 tartrate, and the structure of IN10018 is as follows: 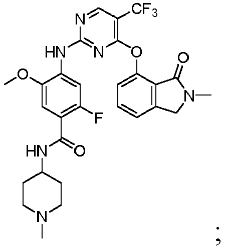 the Defactinib is also known as Difatini, and the CAS number is 1345713-71-4; the CAS number of GSK2256098 is 1224887-10-8; the CAS number of PF-00562271 is 717907-75-0; the CAS number of VS-4718 is 1061353-68-1; the APG-2449 is developed by Yasheng Medicine; and the CAS number of AMP945 is 1393653-34-3.

Optionally, the immunogenic cell death inducer is an RNA polymerase II inhibitor.

Optionally, the RNA polymerase II inhibitor is Lurbinectedin, SEL-120 or a pharmaceutically acceptable salt thereof.

The CAS number of Lurbinectedin is 497871-47-3, and the CAS number of SEL-120 is 1609522-33-9.

Optionally, the RNA polymerase II inhibitor is Lurbinectedin.

Optionally, the immunogenic cell death inducer is an ALK/ROS1 inhibitor.

Optionally, the ALK/ROS1 inhibitor is Crizotinib, SIM-0201, XZP-3621, TQ-B3139, SAF-189s, Ceritinib, Lorlatinib (PF-06463922), Alectinib, Ensartinib, APG-2449, Brigatinib, TQ-B3101, Entrectinib, Repotrectinib or a pharmaceutically acceptable salt thereof, alternatively Crizotinib, Entrectinib or a pharmaceutically acceptable salt thereof.

The CAS number of Crizotinib is 877399-52-5, SIM-0201 is developed by Simcere Pharmaceutical Group, XZP-3621 is developed by Xuanzhu Biopharmaceutical Co., TQ-B3139 is developed by CHIATAI TIANQING Pharmaceutical Company, and Furuitinib (SAF-189s) is jointly developed by Shanghai Institute of Medicine of Chinese Academy of Medical Sciences and Fochon Pharmaceuticals Corporation. The CAS number of Ceritinib is 1032900-25-6, the CAS number of Lorlatinib(PF-06463922) is 1454846-35-5, the CAS number of Alectinib is 1256580-46-7, the CAS number of Ensartinib is 1365267-27-1, APG-2449 is developed by ASCENTAGE PHARMA, the CAS number of Brigatinib is 1197953-54-0, TQ-B3101 is developed by CHIATAI TIANQING Pharmaceutical Company, the CAS number of Entrectinib is 1108743-60-7, and the CAS number of Repotrectinib is 1802220-02-5.

Optionally, the ALK/ROS1 inhibitor is Crizotinib or a pharmaceutically acceptable salt thereof.

Optionally, the immunogenic cell death inducer is a KRAS G12C inhibitor.

Optionally, the KRAS G12C inhibitor is D-1553, ARS-3248, GF-105, JAB-21822, JDQ-443, LY-3537982, Sotorasib (AMG510), Adagrasib (MRTX849) and GDC-6036 or a pharmaceutically acceptable salt thereof, alternatively D-1553, Sotorasib (AMG510) or a pharmaceutically acceptable salt thereof.

D-1553 is developed by InventisBio Co., Ltd., ARS-3248 is developed by Araxes, GF-105 is developed by GenFleet Therapeutics, JAB-21822 is developed by Jacobio Pharma, JDQ-443 is developed by Novartis, the CAS number of LY-3537982 is 2414198-64-2, the CAS number of Sotorasib (AMG510) is 2296729-00-3, the CAS number of Adagrasib (MRTX849) is 2326521-71-3, and the CAS number of GDC-6036 is 2417987-45-0.

Optionally, the KRAS G12C inhibitor is D-1553 or a pharmaceutically acceptable salt thereof.

Optionally, the immunogenic cell death inducer is a KRAS G12D inhibitor.

Optionally, the KRAS G12D inhibitor is MRTX1133, HRS-4642, JAB-22000 or pharmaceutically acceptable salts thereof.

The CAS number of MRTX1133 is 2621928-55-8, JAB-22000 is developed by Jacobio Pharma, and HRS-4642 is developed by Hengrui Pharmaceuticals Co.,Ltd.

Optionally, the KRAS G12D inhibitor is MRTX1133 or a pharmaceutically acceptable salt thereof.

Optionally, the immune checkpoint inhibitor is an anti-PD-1/PD-L1 antibody, a PD-1/PD-L1 small molecule inhibitor or a TIGIT antibody.

Optionally, the immune checkpoint inhibitor is an anti-PD-1/PD-L1 antibody, alternatively, the anti-PD-1/PD-L1 antibody is pembrolizumab, Tislelizumab, Nivolumab, Toripalimab, Atezolizumab, durvalumab, Avelumab, Atezolizumab, Camrelizumab, Sintilimab, Cemiplimab, envafolimab, BMS-936559, JS003, SHR-1316, GS-4224, AN-4005, or MX-10181.

Optionally, the immune checkpoint inhibitor is a PD-1/PD-L1 small molecule inhibitor, alternatively, the PD-1/PD-L1 small molecule inhibitor is INCB-086550, Lazertinib, IMMH-010, CA-170, ABSK043 or RRx-001.

Optionally, the immune checkpoint inhibitor is a TIGIT antibody, alternatively, the TIGIT antibody is Ociperlimab (BGB-A1217), Vibostolimab, domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.

Optionally, the FAK inhibitor is IN10018 or a pharmaceutically acceptable salt thereof, the immunogenic cell death inducer is Lurbinectedin; and the immune checkpoint inhibitor is an anti-PD-1/PD-L1 antibody or a PD-1/PD-L1 small molecule inhibitor.

Optionally, the FAK inhibitor is IN10018 or a pharmaceutically acceptable salt thereof, the immunogenic cell death inducer is Lurbinectedin; and the immune checkpoint inhibitor is an anti-PD-1/PD-L1 antibody.

Optionally, the FAK inhibitor is IN10018 or a pharmaceutically acceptable salt thereof, the immunogenic cell death inducer is Crizotinib or a pharmaceutically acceptable salt thereof; and the immune checkpoint inhibitor is an anti-PD-1/PD-L1 antibody or a PD-1/PD-L1 small molecule inhibitor.

Optionally, the FAK inhibitor is IN10018 or a pharmaceutically acceptable salt thereof, the immunogenic cell death inducer is Crizotinib or a pharmaceutically acceptable salt thereof, and the immune checkpoint inhibitor is an anti-PD-1/PD-L1 antibody.

Optionally, the FAK inhibitor is IN10018 or a pharmaceutically acceptable salt thereof, the immunogenic cell death inducer is D-1553 or a pharmaceutically acceptable salt thereof, and the immune checkpoint inhibitor is an anti-PD-1/PD-L1 antibody or a PD-1/PD-L1 small molecule inhibitor.

Optionally, the FAK inhibitor is IN10018 or a pharmaceutically acceptable salt thereof, the immunogenic cell death inducer is D-1553 or a pharmaceutically acceptable salt thereof; and the immune checkpoint inhibitor is an anti-PD-1/PD-L1 antibody.

Optionally, the FAK inhibitor is IN10018 or a pharmaceutically acceptable salt thereof, the immunogenic cell death inducer is MRTX1133 or a pharmaceutically acceptable salt thereof; and the immune checkpoint inhibitor is an anti-PD-1/PD-L1 antibody or a PD-1/PD-L1 small molecule inhibitor.

Optionally, the FAK inhibitor is IN10018 or a pharmaceutically acceptable salt thereof, the immunogenic cell death inducer is MRTX1133 or a pharmaceutically acceptable salt thereof; and the immune checkpoint inhibitor is a TIGIT antibody.

Optionally, the FAK inhibitor, the immunogenic cell death inducer and the immune checkpoint inhibitor are simultaneously or sequentially administered to the subject.

Optionally, the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML).

Optionally, the tumor is alternatively breast cancer, ovarian cancer, colon cancer (including colorectal cancer), lung cancer (including small cell lung cancer and non-small cell lung cancer), gastric cancer, melanoma, or pancreatic cancer.

Optionally, the tumor is lung cancer or colon cancer (including colorectal cancer) or breast cancer.

### BRIEF DESCRIPTION OF DRAWINGS

In order to more clearly explain the technical solution of examples of the present disclosure, the drawings of examples will be briefly introduced below. Obviously, the drawings in the following description only relate to some examples of the present disclosure, and do not limit the present disclosure.
Fig. 1 shows the curve of inhibitory effect of Lurbinectedin in combination with IN10018 on proliferation of mouse colon cancer CT26 cells versus medicament concentration.
Fig. 2 shows a white light microscope photograph of mouse colon cancer CT26 cells incubated with a medicament for 48 hours.
Fig. 3 shows the percentage of CRT positive cells (3a) and the percentage of Annexin V positive cells (3b) of mouse colon cancer CT26 cells incubated with a medicament for 48 hours.
Fig. 4 shows the curve of inhibitory effect of MRTX1133 in combination with IN10018 on proliferation of mouse colon cancer CT26 cells versus medicament concentration.
Fig. 5 shows a white light microscope photograph of mouse colon cancer CT26 cells incubated with a medicament for 48 hours.
Fig. 6 shows the percentage of CRT positive cells (6a) and the percentage of Annexin V positive cells (6b) of mouse colon cancer CT26 cells incubated with a medicament for 48 hours.
Fig. 7 shows the curve of inhibitory effect of MRTX1133 in combination with IN10018 on the proliferation of mouse lung cancer KPL cells versus medicament concentration.
Fig. 8 shows a white light microscope photograph of mouse lung cancer KPL cells incubated with a medicament for 48 hours.
Fig. 9 shows the percentage of CRT positive cells (9a) and the percentage of Annexin V positive cells (9b) of mouse lung cancer cell KPL cells incubated with a medicament for 48 hours.
Fig. 10 shows the percentage of Calreticulin (CRT) positive cells (10a) and the percentage of Annexin-V positive cells (10b) of mouse colon cancer CT26 cells incubated with a medicament for 48 hours.
Fig. 11 shows the body weight changes of tumor-bearing mice after administration of the assay substance in the mouse colon cancer CT26-KRAS G12C allograft tumor model. The data points represent the average body weight in the group, and the error lines represent the standard error (SEM).
Fig. 12 shows the relative body weight change (%) of tumor-bearing mice after administration of the assay substance in the mouse colon cancer CT26-KRAS G12C allograft tumor model. The relative body weight change was calculated based on the animal's body weight at the beginning of administration. Data points represent the percentage change of average body weight in the group, and error lines represent the standard error (SEM).
Fig. 13 shows the tumor growth curve of tumor-bearing mice after administration of the assay substance in the mouse colon cancer CT26-KRAS G12C allograft tumor model. The data points represent the average tumor volume in the group, and the error lines represent the standard error (SEM).
Fig. 14 shows the tumor growth curve of BALB/c mice subcutaneous allograft tumor model of mouse colorectal cancer CT26 cells after administration. The data points represent the average tumor volume in the group, and the error lines represent the standard error (SEM).
Fig. 15 shows the tumor growth curve of BABL/c mouse subcutaneous allograft tumor model of mouse colorectal cancer CT26 cells after administration. The data points represent the average tumor volume in the group, and the error lines represent the standard error (SEM).
Fig. 16 shows the body weight change curve of BALB/c mouse subcutaneous allograft tumor model of mouse colorectal cancer CT26 cells after administration. The data points represent the average body weight in the group, and the error lines represent the standard error (SEM).
Fig. 17 shows the percentage of Calreticulin (CRT) positive cells (17a) and the percentage of Annexin-V positive cells (17b) of mouse breast cancer 4T1 cells incubated with a medicament for 48 hours.
Fig. 18 shows the percentage of Calreticulin (CRT) positive cells (18a) and the percentage of Annexin-V positive cells (18b) of mouse breast cancer 4T1 cells incubated with a medicament for 48 hours.

### DETAILED DESCRIPTION

In order to make the purpose, technical solutions and advantages of the examples of the present disclosure more clear, the technical solution of the example of the present disclosure will be described clearly and completely with the attached figures. Obviously, the described example is a part of examples of the present disclosure, not the whole examples. Based on the described examples of the present disclosure, all other examples obtained by ordinary people in the field without creative labor belong to the scope of protection of the present disclosure.

The present disclosure can be embodied in other specific forms without departing from the basic attributes of the present disclosure. It should be understood that any and all embodiments of the present disclosure can be combined with the technical features of any other embodiment or a plurality of other embodiments to obtain additional embodiments without conflict. The present disclosure includes further embodiments resulting from such combinations.

All publications and patents mentioned in this disclosure are hereby incorporated by reference in their entirety. If the use or terminology used in any publication and patent incorporated by reference conflicts with the use or terminology used in this disclosure, the use and terminology of the present disclosure shall prevail.

The chapter headings used in this disclosure are only for the purpose of organizing articles and should not be interpreted as limitations on the subject.

Unless otherwise specified, all technical and scientific terms used herein have the usual meaning in the field to which the claimed subject matter belongs. If there are multiple definitions of a term, the definition in the present disclosure shall prevail.

Except in working examples or otherwise indicated, all numbers of quantitative properties such as dosage stated in the description and claims should be understood as being modified in all instances by the term "about". It should also be understood that any numerical range recited in this application is intended to include all subranges within the range and any combination of endpoints of the range or subrange.

"Including", "containing", "comprising" or the like used in this disclosure means that the elements appearing before the word cover the elements listed after the word and their equivalents, but do not exclude elements that are not recorded. The term "including", "containing", or "comprising" used herein can be open, semi-closed and closed. In other words, the term also includes "consisting essentially of" or "consisting of".

### Definition

The following terms and symbols used in this application have the following meanings, unless otherwise specified in the context. As used herein, the term "FAK inhibitor" refers to an effective inhibitor of FAK, which can be suitable for mammals, especially humans. In some embodiments, the FAK inhibitor is IN10018, Defactinib, GSK2256098, PF-00562271, VS-4718, APG-2449, AMP945, AMP886 or pharmaceutically acceptable salts thereof, wherein the structure of IN10018 is 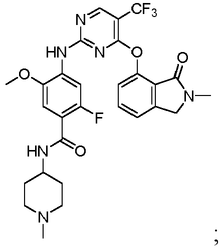 the Defactinib is also known as Difatini, and the CAS number is 1345713-71-4; the CAS number of GSK2256098 is 1224887-10-8; the CAS number of PF-00562271 is 717907-75-0; the CAS number of VS-4718 is 1061353-68-1; the APG-2449 is developed by Yasheng Medicine; the CAS number of AMP945 is 1393653-34-3. In some embodiments, the FAK inhibitor is alternatively IN10018, Defactinib, AMP945 or a pharmaceutically acceptable salt thereof, and in some alternative embodiments, the FAK inhibitor is IN10018 or a pharmaceutically acceptable salt thereof, especially IN10018 tartrate.

The term "immunogenic cell death inducer" as used herein refers to a substance that induces immunogenic cell death (ICD) in an anti-tumor immune response. Immunogenic cell death (ICD) is a specific variant of regulated cell death (RCD), which is driven by stress and can induce adaptive immunity against dead cell antigens. The immunogenic cell death inducer mentioned in present application is an RNA polymerase II inhibitor, an ALK/ROS1 inhibitor, a KRAS G12C inhibitor or a KRAS G12D inhibitor.

In some embodiments, the immunogenic cell death inducer may be an RNA polymerase II inhibitor. In some embodiments, the RNA polymerase II inhibitor is Lurbinectedin, SEL-120 or a pharmaceutically acceptable salt thereof. The CAS number of Lurbinectedin is 497871-47-3, and the CAS number of SEL-120 is 1609522-33-9. In some embodiments, the RNA polymerase II inhibitor is Lurbinectedin.

In some embodiments, the immunogenic cell death inducer is an ALK/ROS1 inhibitor.

In some embodiments, the ALK/ROS1 inhibitor is Crizotinib, SIM-0201, XZP-3621, TQ-B3139, SAF-189s, Ceritinib, Lorlatinib (PF-06463922), Alectinib, Ensartinib, APG-2449, Brigatinib, TQ-B3101, Entrectinib, Repotrectinib or a pharmaceutically acceptable salt thereof, alternatively Crizotinib, Entrectinib or a pharmaceutically acceptable salt thereof.

The CAS number of Crizotinib is 877399-52-5, SIM-0201 is developed by Simcere Pharmaceutical Group, XZP-3621 is developed by Xuanzhu Biopharmaceutical Co., TQ-B3139 is developed by CHIATAI TIANQING Pharmaceutical Company, and Furuitinib (SAF-189s) is jointly developed by Shanghai Institute of Medicine of Chinese Academy of Medical Sciences and Fochon Pharmaceuticals Corporation. The CAS number of Ceritinib is 1032900-25-6, the CAS number of Lorlatinib (PF-06463922) is 1454846-35-5, the CAS number of Alectinib is 1256580-46-7, the CAS number of Ensartinib is 1365267-27-1, APG-2449 is developed by ASCENTAGE PHARMA, the CAS number of Brigatinib is 1197953-54-0, TQ-B3101 is developed by CHIATAI TIANQING Pharmaceutical Company, the CAS number of Entrectinib is 1108743-60-7, and the CAS number of Repotrectinib is 1802220-02-5.

In some embodiments, the ALK/ROS1 inhibitor is Crizotinib or a pharmaceutically acceptable salt thereof.

In some embodiments, the immunogenic cell death inducer is a KRAS G12C inhibitor.

In some embodiments, the KRAS G12C inhibitor is D-1553, ARS-3248, GF-105, JAB-21822, JDQ-443, LY-3537982, Sotorasib (AMG510), Adagrasib (MRTX849), GDC-6036 or a pharmaceutically acceptable salt thereof, alternatively D-1553, Sotorasib (AMG510) or a pharmaceutically acceptable salt thereof.

D-1553 is developed by InventisBio Co., Ltd., ARS-3248 is developed by Araxes, GF-105 is developed by GenFleet Therapeutics, JAB-21822 is developed by Jacobio Pharma, JDQ-443 is developed by Novartis, the CAS number of LY-3537982 is 2414198-64-2, the CAS number of Sotorasib (AMG510) is 2296729-00-3, the CAS number of Adagrasib (MRTX849) is 2326521-71-3, and the CAS number of GDC-6036 is 2417987-45-0.

In some embodiments, the KRAS G12C inhibitor is D-1553 or a pharmaceutically acceptable salt thereof.

In some embodiments, the immunogenic cell death inducer is a KRAS G12D inhibitor.

In some embodiments, the KRAS G12D inhibitor is MRTX1133, HRS-4642, JAB-22000 or a pharmaceutically acceptable salt thereof.

The CAS number of MRTX1133 is 2621928-55-8, JAB-22000 is developed by Jacobio Pharma, and HRS-4642 is developed by Hengrui Pharmaceuticals Co., Ltd.

In some embodiments, the KRAS G12D inhibitor is MRTX1133 or a pharmaceutically acceptable salt thereof.

As used herein, the term "immune checkpoint inhibitor" refers to a medicament that can improve the immune system activity by regulating immune checkpoint pathways (such as PD-1, PDL-1, TIGIT, CTLA-4, LAG-3, TIM-3, etc.). In some embodiments, the immune checkpoint inhibitor is an antagonist of the PD-1/PD-L1 (programmed cell death protein 1) pathway (also called "PD-1 inhibitor") or a TIGIT inhibitor. PD-1 inhibitors are also referred to as PD-1/PD-L1 inhibitors in this disclosure. For example, in the therapeutic methods, medicaments and uses of the present disclosure, the PD-1/PD-L1 inhibitor is a PD-1/PD-L1 antibody, including, but not limited to, pabolizumab (KERIDA/Keytruda/K medicament), Tislelizumab (Baizean), Nivolumab, Toripalimab (Tuoyi), Atezolizumab (Taishengqi), durvalumab (Imfinzi), Avelumab (Bavencio), Atezolizumab (MPDL 3280A/Tecentriq/T medicament), BMS-936559 (fully humanized IgG4 monoclonal antibody against PD-L1), GS-4224, AN-4005 or MX-10181. In some alternative embodiments, the PD-1 inhibitor is Toripalimab. In some embodiments, PD-1 inhibitors are used to treat human subjects. In some embodiments, PD-1 is human PD-1. PD-1/PD-L1 inhibitors also include PD-1/PD-L1 small molecule inhibitors, such as INCB-086550, Lazertinib, IMMH-010, CA-170, ABSK043 or RRx-001.

TIGIT (also known as WUCAM, Vstm3, VSIG9) is a receptor of the Ig superfamily and is a new immune checkpoint besides PD-1/PD-L1. For example, in the therapeutic methods, medicaments and uses of the present disclosure, the immune checkpoint inhibitor is a TIGIT inhibitor, including, but not limited to, Ociperlimab (BGB-A1217), Vibostolimab, domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321. In some embodiments, the TIGIT inhibitors are used to treat human subjects. In order to avoid ambiguity, all antibodies in the present disclosure include double antibodies.

As used herein, "pharmaceutical combination" or "pharmaceutical combination product" can refer to the case of a fixed combination in the form of a dosage unit (for example, all pharmaceutical active ingredients exist in one dosage form) or the case of a product that is administered in combination in a complete kit, and the case of a combination of a medicament and instructions indicating that the medicament can be used in combination with one or more other medicaments.

As used herein, "combination therapy" or "combined medicament" means that a medicament is used in combination with one or more other medicaments to treat a disease, including both the combination of one medicament and one or more other medicaments and the combination of one medicament and instructions indicating that the medicament can be used in combination with one or more other medicaments.

In this application, "simultaneous or sequential administration" refers to the simultaneous administration of two or more medicaments or sequential administration of two or more medicaments at a certain time interval within an administration cycle (for example, within 4 weeks, 3 weeks, 2 weeks, 1 week or 24 hours). The modes of administration (for example, oral administration, intravenous administration, intramuscular administration or subcutaneous administration) may be the same or different, and the frequency/cycle of administration of two or more medicaments may be the same or different. When the therapeutic method, product or use of the present disclosure involves two medicaments, the two medicaments can be administered simultaneously or separately at a certain interval. When the therapeutic method, product or use of the present disclosure involves three medicaments, the three medicaments can be administered at the same time point, or two medicaments can be administered at one time point and the remaining one medicament can be administered at another time point, or each of the three medicaments can be administered at different time points.

In some embodiments, the PD-1/PD-L1 inhibitor is administered intravenously (e.g., as intravenous infusion), subcutaneously or orally. Alternatively, the PD-1/PD-L1 inhibitor is administered by intravenous infusion.

In some embodiments, the TIGIT inhibitor is administered intravenously (e.g., as intravenous infusion), subcutaneously or orally. Alternatively, the TIGIT inhibitor is administered by intravenous infusion.

The ability of immune checkpoint inhibitors to treat cancer depends on the existence of tumor antigen-specific T cells in a tumor tissue. This requires tumor tissues to express antigens that distinguish themselves from their untransformed counterparts, for example, through a new protein product called neoantigen. The neoantigen load of tumor is closely related to immunogenicity and sensitivity (for example, sensitivity to checkpoint inhibitor therapy), which means that tumors with poor immunogenicity should be resistant to these medicaments to a great extent. Therapeutics for releasing tumor antigens that can be ingested by APC, such as those that induce immunogenic cell death (ICD), may promote effective anti-tumor immunity, especially when further combined with a checkpoint inhibitor.

As used herein, the term "treatment" refers to the administration of one or more pharmaceutical substances to a subject suffering from a disease or having symptoms of the disease in order to cure, alleviate, mitigate, change, treat, improve, ameliorate or influence the disease or symptoms of the disease. In some embodiments, the disease is a tumor or cancer.

As used herein, the term "tumor" refers to the abnormal pathological changes formed by the abnormal proliferation of clonal cells caused by the loss of normal regulation of the growth of cells of local tissues at the gene level under the action of various tumorigenic factors. The tumor includes, but is not limited to: bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML). In some embodiments, the tumor is alternatively breast cancer, ovarian cancer, colon cancer (including colorectal cancer), lung cancer (including small cell lung cancer and non-small cell lung cancer), gastric cancer, melanoma or pancreatic cancer; In some embodiments, the tumor is lung cancer, colon cancer (including colorectal cancer) or breast cancer.

As used herein, the term "subject" or "patient" refers to mammals and non-mammals. Mammals refer to any member of mammals, including but not limited to: human; non-human primates, such as chimpanzees and other apes and monkey species; farm animals such as cattle, horses, sheep, goats and pigs; domestic animals such as rabbits, dogs and cats; laboratory animals, including rodents, such as rats, mice and guinea pigs, etc. Examples of non-mammals include but are not limited to birds, etc. The term "subject" is not limited to a specific age or gender. In some embodiments, the subject is a human.

As used herein, the term "pharmaceutically acceptable" means non-toxic, biologically tolerable and suitable for administration to a subject.

As used herein, the term "pharmaceutically acceptable salt" refers to non-toxic and biologically tolerable acid addition salts suitable for administration to subjects, including but not limited to: acid addition salts formed with inorganic acids, such as hydrochloride, hydrobromide, carbonate, bicarbonate, phosphate, sulfate, sulfite, nitrate, etc.; and acid addition salts with organic acids, such as formate, acetate, malate, maleate, fumarate, tartrate, succinate, citrate, lactate, methanesulfonate, p-toluenesulfonate, 2-hydroxyethyl sulfonate, benzoate, salicylate, stearate and salts formed with alkanedicarboxylic acids of formula HOOC-(CH₂)ₙ-COOH (where n is 0-4), etc.

Furthermore, a pharmaceutically acceptable acid addition salt can be obtained by dissolving a free base in a suitable solvent and treating the solution with an acid according to a conventional operation for preparing an acid addition salt from a basic compound. Those skilled in the art can determine various synthetic methods that can be used to prepare nontoxic pharmaceutically acceptable acid addition salts without redundant experiments.

As used herein, the term "pharmaceutically acceptable composition" means that it must be chemically and/or toxicologically compatible with other ingredients included in the preparation, and/or compatible with the subject to be treated. As used herein, the term "therapeutically effective amount" refers to an amount generally sufficient to produce a beneficial therapeutic effect on a subject. The therapeutically effective amount of the present disclosure can be determined by conventional methods (such as modeling, dose escalation study or clinical trial) combined with conventional influencing factors (such as administration mode, pharmacokinetics of compounds, severity and course of diseases, medical history of subjects, health status of subjects, response degree of subjects to medicaments, etc.).

As used herein, the term "inhibition" refers to a decrease in the baseline activity of a biological activity or process.

As used herein, the term "kit" refers to a box for containing chemical reagents for detecting chemical components, medicament residues, virus types and the like. The kit of the present disclosure can include (i) one, two or three of an FAK inhibitor, an immunogenic cell death inducer and an immune checkpoint inhibitor; and (ii) an instruction indicating that the FAK inhibitor, the immunogenic cell death inducer and the immune checkpoint inhibitor can be used to treat tumors in the subject; wherein the immunogenic cell death inducer is an RNA polymerase II inhibitor, an ALK/ROS1 inhibitor, a KRAS G12C inhibitor or a KRAS G12D inhibitor. In one embodiment, the kit comprises (i) an FAK inhibitor; and (ii) an instruction indicating that the FAK inhibitor, the immunogenic cell death inducer and the immune checkpoint inhibitor can be used to treat tumors in a subject. In one embodiment, the kit includes (i) an immunogenic cell death inducer; and (ii) an instruction indicating that the FAK inhibitor, the immunogenic cell death inducer and the immune checkpoint inhibitor can be used to treat tumors in a subject. In one embodiment, the kit comprises (i) an immune checkpoint inhibitor; and (ii) an instruction indicating that the FAK inhibitor, the immunogenic cell death inducer and the immune checkpoint inhibitor can be used to treat tumors in a subject. In one embodiment, the kit comprises (i) an FAK inhibitor, an immunogenic cell death inducer and an immune checkpoint inhibitor; and (ii) an instruction indicating that the FAK inhibitor, the immunogenic cell death inducer and the immune checkpoint inhibitor can be used to treat tumors in a subject. In one embodiment, the kit comprises (i) an FAK inhibitor; and (ii) an instruction indicating that the FAK inhibitor and the immunogenic cell death inducer can be used to treat tumors in a subject. In one embodiment, the kit includes (i) an immunogenic cell death inducer; and (ii) an instruction indicating that the immunogenic cell death inducer and the FAK inhibitor can be used to treat tumors in a subject.

The compounds of a kit can be contained in separate containers. Optionally, two or more compounds are contained in the same container. For example, the kit may include a first container, a second container, a third container, and a package insert, wherein the first container includes at least one dose of a medicament including an FAK inhibitor, the second container includes at least one dose of an immunogenic cell death inducer, and the third container includes at least one dose of a medicament including an immune checkpoint inhibitor, and the package insert includes instructions of treating a tumor in a subject with the medicament. The first container, the second container and the third container may contain the same or different shapes (e.g., vials, syringes and bottles) and/or materials (e.g., plastic or glass). The kit can also include other materials that can help to administer medicaments, such as diluents, filters, IV bags and lines, needles and syringes.

The exact amount of the FAK inhibitor, the immunogenic cell death inducer and the immune checkpoint inhibitor to be administered to a subject will depend on various factors, such as a given medicament or compound, pharmaceutical preparation, route of administration, type of disease, symptoms, identity of the subject or host to be treated, etc., but it can still be routinely determined by those skilled in the art. For example, determining the effective amount also depends on the degree, severity and type of cell proliferation. The skilled person will be able to determine the appropriate dosage based on these and other factors.

The FAK inhibitor, the immunogenic cell death inducer and the immune checkpoint inhibitor can be administered in an appropriate manner, such as oral administration, intravenous administration, intramuscular administration, or subcutaneous administration.

For example, when administered orally, the medicament can be administered orally together with a pharmaceutically acceptable carrier such as an inert diluent or an absorbable edible carrier. They can be encapsulated in hard-shell or soft-shell gelatin capsules, can be pressed into tablets, or can be directly mixed with patients' food. For example, medicaments can be combined with one or more excipients and used in the form of ingestible tablets, oral tablets, lozenges, capsules, elixirs, suspensions, syrups or wafers. Tablets, lozenges, pills, capsules, and the like may further include a binder, such as tragacanth gum, arabic gum, corn starch or gelatin; an excipient, such as dicalcium phosphate; a disintegrator, such as corn starch, potato starch, alginic acid, and the like; a lubricant, such as magnesium stearate; or a sweetener, such as sucrose, fructose, lactose or aspartame; or a flavoring agent.

For example, when administered intravenously or intraperitoneally by infusion or injection, the medicament solution can be prepared in water, optionally mixed with a non-toxic surfactant.

Exemplary pharmaceutical dosage forms for injection or infusion include a sterile aqueous solution, a dispersion, or a sterile powder containing an active ingredient, which is suitable for temporarily preparing a sterile injection or infusion solution or dispersion. In any case, the final dosage form should be sterile, fluid and stable under the conditions of production and storage.

Sterile injection solution can be prepared by mixing the required amount of medicaments with various other components mentioned above into a suitable solvent, and then filtering and sterilizing the mixture. For sterile powder used to prepare sterile injection solution, the alternative preparation methods can be vacuum drying and freeze drying techniques, which can produce powder of active ingredient plus any other required ingredients existing after previous sterile filtration.

The amount of the FAK inhibitor, the immunogenic cell death inducer and the immune checkpoint inhibitor required for treatment can vary not only with the specific reagents selected, but also with the route of administration, the nature of the diseases to be treated, and the age and condition of the patients, and can be finally decided by the attending physician or clinician. However, in general, the dosage can be in the range of about 0.1 to about 50mg/kg body weight per day.

The FAK inhibitor is administered in a dosage range of 5mg/day to 500 mg/day in adults. In a specific embodiment, IN10018 or a pharmaceutically acceptable salt thereof is administered at a dose of 5 mg/day to 100 mg/day in adults, for example, IN10018 or a pharmaceutically acceptable salt thereof is administered at a dose of 25 mg/day to 100 mg/day in adults, and the dose is calculated as free base.

The dosage range of the immunogenic cell death inducer in adults depends on each medicament in the dosage range of adults. In some embodiments, the RNA polymerase II inhibitor is administered in a dosage range of 1-25 mg/m² every 21 days (treatment cycle) in adults. In some specific embodiments, Lurbinectedin is administered in a dosage range of 1-5 mg/m² every 21 days in adults. In some embodiments, the ALK/ROS1 inhibitor is administered in a dosage range of 2-1000 mg per day in adults. In some specific embodiments, Crizotinib is administered in a dosage range of 50-500 mg per day in adults. In some specific embodiments, the KRAS G12C inhibitor is administered in a dosage range of 10-500 mg per day in adults. In some specific embodiments, D-1553 is administered in a dosage range of 10-250 mg per day in adults. In some embodiments, the KRAS G12D inhibitor is administered in a dosage range of 25-800 mg per day in adults. In some specific embodiments, MRTX1133 is administered in a dosage range of 25-600 mg per day in adults. All the above amounts are based on free base.

The immune checkpoint inhibitor is administered each time at a dose of 2-10mg/kg or 50-1200mg for adults, and once every 2 to 3 weeks. In a specific embodiment, the immune checkpoint inhibitor is administered each time at a dose of 3-10mg/kg or 100-1200mg for adults and once every two to three weeks.

Technical and scientific terms not specifically defined used herein have the meanings commonly understood by those skilled in the art to which this disclosure belongs.

In some embodiments, the present disclosure also discloses:
1. An FAK inhibitor, an immunogenic cell death inducer and an immune checkpoint inhibitor, which are used in a method for treating tumors in a subject, wherein the immunogenic cell death inducer is an RNA polymerase II inhibitor, an ALK/ROS1 inhibitor, a KRAS G12C inhibitor or a KRAS G12D inhibitor.
2. The FAK inhibitor, the immunogenic cell death inducer and the immune checkpoint inhibitor according to embodiment 1, wherein the FAK inhibitor is IN10018, Defactinib, GSK2256098, PF-00562271, VS-4718, APG-2449, AMP945, AMP886 or a pharmaceutically acceptable salt thereof, alternatively IN10018, Defactinib, AMP945 or a pharmaceutically acceptable salt thereof, yet alternatively IN10018 or a pharmaceutically acceptable salt thereof, alternatively IN10018 tartrate, and the structure of IN10018 is: 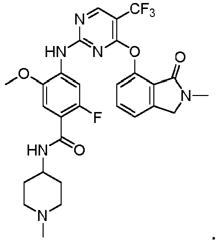
3. The FAK inhibitor, the immunogenic cell death inducer and the immune checkpoint inhibitor according to embodiment 1 or 2, wherein the immunogenic cell death inducer is an RNA polymerase II inhibitor.
4. The FAK inhibitor, the immunogenic cell death inducer and the immune checkpoint inhibitor according to any one of embodiments 1-3, wherein the RNA polymerase II inhibitor is Lurbinectedin, SEL-120 or a pharmaceutically acceptable salt thereof.
5. The FAK inhibitor, the immunogenic cell death inducer and the immune checkpoint inhibitor according to any one of embodiments 3-4, wherein the RNA polymerase II inhibitor is Lurbinectedin.
6. The FAK inhibitor, the immunogenic cell death inducer and the immune checkpoint inhibitor according to any one of embodiments 1-2, wherein the immunogenic cell death inducer is an ALK/ROS1 inhibitor.
7. The FAK inhibitor, the immunogenic cell death inducer and the immune checkpoint inhibitor according to embodiment 6, wherein the ALK/ROS1 inhibitor is Crizotinib, SIM-0201, XZP-3621, TQ-B3139, SAF-189s, Ceritinib, Lorlatinib (PF-06463922), Alectinib, Ensartinib, APG-2449, Brigatinib, TQ-B3101, Entrectinib, Repotrectinib or a pharmaceutically acceptable salt thereof, alternatively Crizotinib, Entrectinib or a pharmaceutically acceptable salt thereof.
8. The FAK inhibitor, the immunogenic cell death inducer and the immune checkpoint inhibitor according to any one of embodiments 6-7, wherein the ALK/ROS1 inhibitor is Crizotinib or a pharmaceutically acceptable salt thereof.
9. The FAK inhibitor, the immunogenic cell death inducer and the immune checkpoint inhibitor according to any one of embodiments 1-2, wherein the immunogenic cell death inducer is a KRAS G12C inhibitor.
10. The FAK inhibitor, the immunogenic cell death inducer and the immune checkpoint inhibitor according to embodiment 9, wherein the KRAS G12C inhibitor is D-1553, ARS-3248, GF-105, JAB-21822, JDQ-443, LY-3537982, Sotorasib (AMG510), Adagrasib (MRTX849) or GDC-6036 or a pharmaceutically acceptable salt thereof, alternatively D-1553, Sotorasib (AMG510) or a pharmaceutically acceptable salt thereof.
11. The FAK inhibitor, the immunogenic cell death inducer and the immune checkpoint inhibitor according to any one of embodiments 9-10, wherein the KRAS G12C inhibitor is D-1553 or a pharmaceutically acceptable salt thereof.
12. The FAK inhibitor, the immunogenic cell death inducer and the immune checkpoint inhibitor according to any one of embodiments 1-2, wherein the immunogenic cell death inducer is a KRAS G12D inhibitor.
13. The FAK inhibitor, the immunogenic cell death inducer and the immune checkpoint inhibitor according to embodiment 12, wherein the KRAS G12D inhibitor is MRTX1133, HRS-4642, JAB-22000 or a pharmaceutically acceptable salt thereof.
14. The FAK inhibitor, the immunogenic cell death inducer and the immune checkpoint inhibitor according to any one of embodiments 12-13, wherein the KRAS G12D inhibitor is MRTX1133 or a pharmaceutically acceptable salt thereof.
15. The FAK inhibitor, the immunogenic cell death inducer and the immune checkpoint inhibitor according to any one of embodiments 1 to 14, wherein the immune checkpoint inhibitor is an anti-PD-1/PD-L1 antibody, a PD-1/PD-L1 small molecule inhibitor or a TIGIT antibody.
16. The FAK inhibitor, the immunogenic cell death inducer and the immune checkpoint inhibitor according to any one of embodiments 1 to 15, wherein the immune checkpoint inhibitor is an anti-PD-1/PD-L1 antibody, and alternatively the anti-PD-1/PD-L1 antibody is pembrolizumab, Tislelizumab, Nivolumab, Toripalimab, Atezolizumab, durvalumab, Avelumab, Atezolizumab, Camrelizumab, Sintilimab, Cemiplimab, envafolimab, BMS-936559, JS003, SHR-1316, GS-4224, AN-4005, or MX-10181.
17. The FAK inhibitor, the immunogenic cell death inducer and the immune checkpoint inhibitor according to any one of embodiments 1-15, wherein the immune checkpoint inhibitor is a PD-1/PD-L1 small molecule inhibitor, and alternatively the PD-1/PD-L1 small molecule inhibitor is INCB-086550, Lazertinib, IMMH-010, CA-170, ABSK043 or RRx-001.
18. The FAK inhibitor, the immunogenic cell death inducer and the immune checkpoint inhibitor according to any one of embodiments 1 to 15, wherein the immune checkpoint inhibitor is a TIGIT antibody, and alternatively, the TIGIT antibody is Ociperlimab (BGB-A1217), Vibostolimab, domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
19. The FAK inhibitor, the immunogenic cell death inducer and the immune checkpoint inhibitor according to embodiment 1, wherein the FAK inhibitor is IN10018 or a pharmaceutically acceptable salt thereof, the immunogenic cell death inducer is Lurbinectedin, and the immune checkpoint inhibitor is an anti-PD-1/PD-L1 antibody or a PD-1/PD-L1 small molecule inhibitor, and alternatively, the immune checkpoint inhibitor is an anti-PD-1/PD-L1 antibody.
20. The FAK inhibitor, the immunogenic cell death inducer and the immune checkpoint inhibitor according to embodiment 1, wherein the FAK inhibitor is IN10018 or a pharmaceutically acceptable salt thereof, the immunogenic cell death inducer is Crizotinib or a pharmaceutically acceptable salt thereof, and the immune checkpoint inhibitor is an anti-PD-1/PD-L1 antibody or a PD-1/PD-L1 small molecule inhibitor, and alternatively, the immune checkpoint inhibitor is an anti-PD-1/PD-L1 antibody.
21. The FAK inhibitor, the immunogenic cell death inducer and the immune checkpoint inhibitor according to embodiment 1, wherein the FAK inhibitor is IN10018 or a pharmaceutically acceptable salt thereof, the immunogenic cell death inducer is D-1553 or a pharmaceutically acceptable salt thereof, and the immune checkpoint inhibitor is an anti-PD-1/PD-L1 antibody or a PD-1/PD-L1 small molecule inhibitor, and alternatively, the immune checkpoint inhibitor is an anti-PD-1/PD-L1 antibody.
22. The FAK inhibitor, the immunogenic cell death inducer and the immune checkpoint inhibitor according to embodiment 1, wherein the FAK inhibitor is IN10018 or a pharmaceutically acceptable salt thereof, the immunogenic cell death inducer is MRTX1133 or a pharmaceutically acceptable salt thereof, and the immune checkpoint inhibitor is a TIGIT antibody.
23. The FAK inhibitor, the immunogenic cell death inducer and the immune checkpoint inhibitor according to any one of embodiments 1 to 22, wherein the FAK inhibitor, the immunogenic cell death inducer and the immune checkpoint inhibitor are administered simultaneously or sequentially to the subject.
24. The FAK inhibitor, the immunogenic cell death inducer and the immune checkpoint inhibitor according to any one of embodiments 1 to 23, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
25. The FAK inhibitor, the immunogenic cell death inducer and the immune checkpoint inhibitor according to embodiment 24, wherein the tumor is lung cancer, colon cancer (including colorectal cancer) or breast cancer.
26. A kit or a pharmaceutically acceptable composition, comprising:
   (a) an FAK inhibitor;
   (b) an immunogenic cell death inducer; and
   (c) an immune checkpoint inhibitor,
   wherein the immunogenic cell death inducer is an RNA polymerase II inhibitor, an ALK/ROS1 inhibitor, a KRAS G12C inhibitor or a KRAS G12D inhibitor.
27. The kit or composition according to embodiment 26, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945 or a pharmaceutically acceptable salt thereof, and yet alternatively, the FAK inhibitor is IN10018, or a pharmaceutically acceptable salt thereof, alternatively, the FAK inhibitor is IN10018 tartrate, wherein the IN10018 has a structure of: 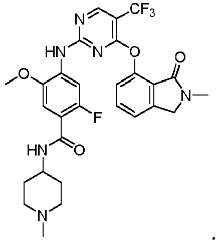
28. The kit or composition according to any one of embodiments 26-27, wherein the immunogenic cell death inducer is an RNA polymerase II inhibitor.
29. The kit or composition according to embodiment 28, wherein the RNA polymerase II inhibitor is Lurbinectedin, SEL-120, or a pharmaceutically acceptable salt thereof.
30. The kit or composition according to any one of embodiments 28-29, wherein the RNA polymerase II inhibitor is Lurbinectedin.
31. The kit or composition according to any one of embodiments 26-27, wherein the immunogenic cell death inducer is an ALK/ROS1 inhibitor.
32. The kit or composition according to embodiment 31, wherein the ALK/ROS1 inhibitor is Crizotinib, SIM-0201, XZP-3621, TQ-B3139, SAF-189s, Ceritinib, Lorlatinib (PF-06463922), Alectinib, Ensartinib, APG-2449, Brigatinib, TQ-B3101, Entrectinib, Repotrectinib, or a pharmaceutically acceptable salt thereof, alternatively, the ALK/ROS1 inhibitor is Crizotinib, Entrectinib, or a pharmaceutically acceptable salt thereof.
33. The kit or composition according to any one of embodiments 31-32, wherein the ALK/ROS1 inhibitor is Crizotinib, or a pharmaceutically acceptable salt thereof.
34. The kit or composition according to any one of embodiments 26-27, wherein the immunogenic cell death inducer is a KRAS G12C inhibitor.
35. The kit or composition according to embodiment 34, wherein the KRAS G12C inhibitor is D-1553, ARS-3248, GF-105, JAB-21822, JDQ-443, LY-3537982, Sotorasib (AMG510), Adagrasib (MRTX849), GDC-6036, or a pharmaceutically acceptable salt thereof, alternatively, the KRAS G12C inhibitor is D-1553, Sotorasib (AMG510), or a pharmaceutically acceptable salt thereof.
36. The kit or composition according to any one of embodiments 34-35, wherein the KRAS G12C inhibitor is D-1553, or a pharmaceutically acceptable salt thereof.
37. The kit or composition according to any one of embodiments 26-27, wherein the immunogenic cell death inducer is a KRAS G12D inhibitor.
38. The kit or composition according to embodiment 37, wherein the KRAS G12D inhibitor is MRTX1133, HRS-4642, JAB-22000, or a pharmaceutically acceptable salt thereof.
39. The kit or composition according to any one of embodiments 37-38, wherein the KRAS G12D inhibitor is MRTX1133, or a pharmaceutically acceptable salt thereof.
40. The kit or composition according to any one of embodiments 26-39, wherein the immune checkpoint inhibitor is an anti-PD-1/PD-L1 antibody, a PD-1/PD-L1 small molecule inhibitor, or a TIGIT antibody.
41. The kit or composition according to any one of embodiments 26-40, wherein the immune checkpoint inhibitor is an anti-PD-1/PD-L1 antibody, alternatively, the anti-PD-1/PD-L1 antibody is pembrolizumab, tislelizumab, nivolumab, toripalimab, atezolizumab, durvalumab, avelumab, atezolizumab, camrelizumab, sintilimab, cemiplimab, envafolimab, BMS-936559, JS003, SHR-1316, GS-4224, AN-4005, or MX-10181.
42. The kit or composition according to any one of embodiments 26-40, wherein the immune checkpoint inhibitor is the PD-1/PD-L1 small molecule inhibitor, alternatively, the PD-1/PD-L1 small molecule inhibitor is INCB-086550, Lazertinib, IMMH-010, CA-170, ABSK043, or RRx-001.
43. The kit or composition according to any one of embodiments 26-40, wherein the immune checkpoint inhibitor is a TIGIT antibody, alternatively, the TIGIT antibody is ociperlimab (BGB-A1217), vibostolimab, domvanalimab (AB154), tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
44. The kit or composition according to embodiment 26, wherein the FAK inhibitor is IN10018 or a pharmaceutically acceptable salt thereof, the immunogenic cell death inducer is Lurbinectedin; and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.
45. The kit or composition according to embodiment 26, wherein the FAK inhibitor is IN10018 or a pharmaceutically acceptable salt thereof, the immunogenic cell death inducer is Crizotinib or a pharmaceutically acceptable salt thereof, and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.
46. The kit or composition according to embodiment 26, wherein the FAK inhibitor is IN10018 or a pharmaceutically acceptable salt thereof, the immunogenic cell death inducer is D-1553 or a pharmaceutically acceptable salt thereof, and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.
47. The kit or composition according to embodiment 26, wherein the FAK inhibitor is IN10018 or a pharmaceutically acceptable salt thereof, the immunogenic cell death inducer is MRTX1133 or a pharmaceutically acceptable salt thereof, and the immune checkpoint inhibitor is a TIGIT antibody.
48. The kit or composition according to any one of embodiments 26-47, wherein the kit or composition is used as a medicament.
49. The kit or composition according to embodiment 48, wherein the medicament is used for treating tumors, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
50. The kit or composition according to embodiment 49, wherein the tumor is lung cancer, colon cancer (including colorectal cancer) or breast cancer.
51. A method for treating tumors in a subject, wherein the method comprises administering to the subject a therapeutically effective amount of an FAK inhibitor, an immunogenic cell death inducer and an immune checkpoint inhibitor, wherein the immunogenic cell death inducer is an RNA polymerase II inhibitor, an ALK/ROS1 inhibitor, a KRAS G12C inhibitor, or a KRAS G12D inhibitor.
52. The method according to embodiment 51, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945 or a pharmaceutically acceptable salt thereof, yet alternatively, the FAK inhibitor is IN10018, or a pharmaceutically acceptable salt thereof, alternatively, the FAK inhibitor is IN10018 tartrate, wherein the IN10018 has a structure of: 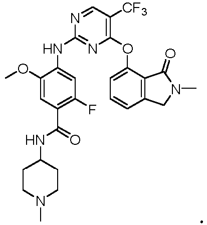
53. The method according to any one of embodiments 51-52, wherein the immunogenic cell death inducer is an RNA polymerase II inhibitor.
54. The method according to embodiment 53, wherein the RNA polymerase II inhibitor is Lurbinectedin, SEL-120, or a pharmaceutically acceptable salt thereof.
55. The method according to any one of embodiments 53-54, wherein the RNA polymerase II inhibitor is Lurbinectedin.
56. The method according to any one of embodiments 51-52, wherein the immunogenic cell death inducer is an ALK/ROS1 inhibitor.
57. The method according to embodiment 56, wherein the ALK/ROS1 inhibitor is Crizotinib, SIM-0201, XZP-3621, TQ-B3139, SAF-189s, Ceritinib, Lorlatinib (PF-06463922), Alectinib, Ensartinib, APG-2449, Brigatinib, TQ-B3101, Entrectinib, Repotrectinib, or a pharmaceutically acceptable salt thereof, alternatively, the ALK/ROS1 inhibitor is Crizotinib, Entrectinib, or a pharmaceutically acceptable salt thereof.
58. The method according to any one of embodiments 56-57, wherein the ALK/ROS1 inhibitor is Crizotinib or a pharmaceutically acceptable salt thereof.
59. The method according to any one of embodiments 51-52, wherein the immunogenic cell death inducer is a KRAS G12C inhibitor.
60. The method according to embodiment 59, wherein the KRAS G12C inhibitor is D-1553, ARS-3248, GF-105, JAB-21822, JDQ-443, LY-3537982, Sotorasib (AMG510), Adagrasib (MRTX849), GDC-6036, or a pharmaceutically acceptable salt thereof, alternatively, the KRAS G12C inhibitor is D-1553, Sotorasib (AMG510), or a pharmaceutically acceptable salt thereof.
61. The method according to any one of embodiments 59-60, wherein the KRAS G12C inhibitor is D-1553, or a pharmaceutically acceptable salt thereof.
62. The method according to any one of embodiments 51-52, wherein the immunogenic cell death inducer is a KRAS G12D inhibitor.
63. The method according to embodiment 62, wherein the KRAS G12D inhibitor is MRTX1133, HRS-4642, JAB-22000, or a pharmaceutically acceptable salt thereof.
64. The method according to any one of embodiments 62-63, wherein the KRAS G12D inhibitor is MRTX1133, or a pharmaceutically acceptable salt thereof.
65. The method according to any one of embodiments 51-64, wherein the immune checkpoint inhibitor is an anti-PD-1/PD-L1 antibody, a PD-1/PD-L1 small molecule inhibitor or a TIGIT antibody.
66. The method according to embodiment 65, wherein the immune checkpoint inhibitor is an anti-PD-1/PD-L1 antibody, alternatively, the anti-PD-1/PD-L1 antibody is pembrolizumab, tislelizumab, nivolumab, toripalimab, atezolizumab, durvalumab, avelumab, Atezolizumab, camrelizumab, sintilimab, cemiplimab, envafolimab, BMS-936559, JS003, SHR-1316, GS-4224, AN-4005, or MX-10181.
67. The method according to embodiment 65, wherein the immune checkpoint inhibitor is a PD-1/PD-L1 small molecule inhibitor, alternatively, the PD-1/PD-L1 small molecule inhibitor is INCB-086550, Lazertinib, IMMH-010, CA-170, ABSK043, or RRx-001.
68. The method according to embodiment 65, wherein the immune checkpoint inhibitor is a TIGIT antibody, alternatively, the TIGIT antibody is Ociperlimab(BGB-A1217), vibostolimab, domvanalimab (AB154), tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
69. The method according to embodiment 51, wherein the FAK inhibitor is IN10018 or a pharmaceutically acceptable salt thereof, the immunogenic cell death inducer is Lurbinectedin; and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.
70. The method according to embodiment 51, wherein the FAK inhibitor is IN10018 or a pharmaceutically acceptable salt thereof, the immunogenic cell death inducer is Crizotinib or a pharmaceutically acceptable salt thereof, and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.
71. The method according to embodiment 51, wherein the FAK inhibitor is IN10018 or a pharmaceutically acceptable salt thereof, the immunogenic cell death inducer is D-1553 or a pharmaceutically acceptable salt thereof, and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.
72. The method according to embodiment 51, wherein the FAK inhibitor is IN10018 or a pharmaceutically acceptable salt thereof, the immunogenic cell death inducer is MRTX1133 or a pharmaceutically acceptable salt thereof, and the immune checkpoint inhibitor is an anti-PD-1/PD-L1 antibody, or a PD-1/PD-L1 small molecule inhibitor, alternatively, the immune checkpoint inhibitor is a TIGIT antibody.
73. The method according to any one of embodiments 51-72, wherein the FAK inhibitor, the immunogenic cell death inducer and the immune checkpoint inhibitor are administered simultaneously or sequentially to the subject.
74. The method according to any one of embodiments 51-73, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
75. The method according to embodiment 74, wherein the tumor is breast cancer, ovarian cancer, or colon cancer (including colorectal cancer).
76. An FAK inhibitor, an immunogenic cell death inducer and an immune checkpoint inhibitor, which are used in a method of treating a tumor by increasing immunogenic cell death in a subject, wherein the immunogenic cell death inducer is an RNA polymerase II inhibitor, an ALK/ROS1 inhibitor, a KRAS G12C inhibitor or a KRAS G12D inhibitor.
77. The FAK inhibitor, the immunogenic cell death inducer and the immune checkpoint inhibitor according to embodiment 76, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, and yet alternatively, the FAK inhibitor is IN10018, or a pharmaceutically acceptable salt thereof, alternatively, the FAK inhibitor is IN10018 tartrate, wherein the IN10018 has a structure of: 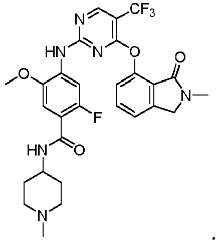
78. The FAK inhibitor, the immunogenic cell death inducer and the immune checkpoint inhibitor according to any one of embodiments 76-77, wherein the immunogenic cell death inducer is an RNA polymerase II inhibitor.
79. The FAK inhibitor, the immunogenic cell death inducer and the immune checkpoint inhibitor according to embodiment 78, wherein the RNA polymerase II inhibitor is Lurbinectedin, SEL-120, or a pharmaceutically acceptable salt thereof.
80. The FAK inhibitor, the immunogenic cell death inducer and the immune checkpoint inhibitor according to any one of embodiments 78-79, wherein the RNA polymerase II inhibitor is Lurbinectedin.
81. The FAK inhibitor, the immunogenic cell death inducer and the immune checkpoint inhibitor according to any one of embodiments 76-77, wherein the immunogenic cell death inducer is an ALK/ROS1 inhibitor.
82. The FAK inhibitor, the immunogenic cell death inducer and the immune checkpoint inhibitor according to embodiment 81, wherein the ALK/ROS1 inhibitor is Crizotinib, SIM-0201, XZP-3621, TQ-B3139, SAF-189s, Ceritinib, Lorlatinib (PF-06463922), Alectinib, Ensartinib, APG-2449, Brigatinib, TQ-B3101, Entrectinib, Repotrectinib, or a pharmaceutically acceptable salt thereof, alternatively, the ALK/ROS1 inhibitor is Crizotinib, Entrectinib, or a pharmaceutically acceptable salt thereof.
83. The FAK inhibitor, the immunogenic cell death inducer and the immune checkpoint inhibitor according to any one of embodiments 81-82, wherein the ALK/ROS1 inhibitor is Crizotinib, or a pharmaceutically acceptable salt thereof.
84. The FAK inhibitor, the immunogenic cell death inducer and the immune checkpoint inhibitor according to any one of embodiments 76-77, wherein the immunogenic cell death inducer is a KRAS G12C inhibitor.
85. The FAK inhibitor, the immunogenic cell death inducer and the immune checkpoint inhibitor according to embodiment 84, wherein the KRAS G12C inhibitor is D-1553, ARS-3248, GF-105, JAB-21822, JDQ-443, LY-3537982, Sotorasib (AMG510), Adagrasib (MRTX849), GDC-6036, or a pharmaceutically acceptable salt thereof, alternatively, the KRAS G12C inhibitor is D-1553, Sotorasib (AMG510), or a pharmaceutically acceptable salt thereof.
86. The FAK inhibitor, the immunogenic cell death inducer and the immune checkpoint inhibitor according to any one of embodiments 84-85, wherein the KRAS G12C inhibitor is D-1553, or a pharmaceutically acceptable salt thereof.
87. The FAK inhibitor, the immunogenic cell death inducer and the immune checkpoint inhibitor according to any one of embodiments 76-77, wherein the immunogenic cell death inducer is a KRAS G12D inhibitor.
88. The FAK inhibitor, the immunogenic cell death inducer and the immune checkpoint inhibitor according to embodiment 87, wherein the KRAS G12D inhibitor is MRTX1133, HRS-4642, JAB-22000, or a pharmaceutically acceptable salt thereof.
89. The FAK inhibitor, the immunogenic cell death inducer and the immune checkpoint inhibitor according to any one of embodiments 87-88, wherein the KRAS G12D inhibitor is MRTX1133, or a pharmaceutically acceptable salt thereof.
90. The FAK inhibitor, the immunogenic cell death inducer and the immune checkpoint inhibitor according to any one of embodiments 76-89, wherein the immune checkpoint inhibitor is an anti-PD-1/PD-L1 antibody, a PD-1/PD-L1 small molecule inhibitor, or a TIGIT antibody.
91. The FAK inhibitor, the immunogenic cell death inducer and the immune checkpoint inhibitor according to any one of embodiments 76-90, wherein the immune checkpoint inhibitor is an anti-PD-1/PD-L1 antibody, alternatively, the anti-PD-1/PD-L1 antibody is pembrolizumab, tislelizumab, nivolumab, toripalimab, atezolizumab, durvalumab, avelumab, atezolizumab, camrelizumab, sintilimab, cemiplimab, envafolimab, BMS-936559, JS003, SHR-1316, GS-4224, AN-4005, or MX-10181.
92. The FAK inhibitor, the immunogenic cell death inducer and the immune checkpoint inhibitor according to any one of embodiments 76-90, wherein the immune checkpoint inhibitor is a PD-1/PD-L1 small molecule inhibitor, alternatively, the PD-1/PD-L1 small molecule inhibitor is INCB-086550, Lazertinib, IMMH-010, CA-170, ABSK043, or RRx-001.
93. The FAK inhibitor, the immunogenic cell death inducer and the immune checkpoint inhibitor according to any one of embodiments 76-90, wherein the immune checkpoint inhibitor is a TIGIT antibody, alternatively, the TIGIT antibody is ociperlimab (BGB-A1217), vibostolimab, domvanalimab (AB154), tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
94. The FAK inhibitor, the immunogenic cell death inducer and the immune checkpoint inhibitor according to embodiment 76, wherein the FAK inhibitor is IN10018 or a pharmaceutically acceptable salt thereof, the immunogenic cell death inducer is Lurbinectedin; and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.
95. The FAK inhibitor, the immunogenic cell death inducer and the immune checkpoint inhibitor according to embodiment 76, wherein the FAK inhibitor is IN10018 or a pharmaceutically acceptable salt thereof, the immunogenic cell death inducer is Crizotinib or a pharmaceutically acceptable salt thereof, and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.
96. The FAK inhibitor, the immunogenic cell death inducer and the immune checkpoint inhibitor according to embodiment 76, wherein the FAK inhibitor is IN10018 or a pharmaceutically acceptable salt thereof, the immunogenic cell death inducer is D-1553 or a pharmaceutically acceptable salt thereof, and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.
97. The FAK inhibitor, the immunogenic cell death inducer and the immune checkpoint inhibitor according to embodiment 76, wherein the FAK inhibitor is IN10018 or a pharmaceutically acceptable salt thereof, the immunogenic cell death inducer is MRTX1133 or a pharmaceutically acceptable salt thereof, and the immune checkpoint inhibitor is a TIGIT antibody.
98. The FAK inhibitor, the immunogenic cell death inducer and the immune checkpoint inhibitor according to any one of embodiments 76-97, wherein the FAK inhibitor, the immunogenic cell death inducer and the immune checkpoint inhibitor are administered simultaneously or sequentially to the subject.
99. The FAK inhibitor, the immunogenic cell death inducer and the immune checkpoint inhibitor according to any one of embodiments 76-98, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
100. The FAK inhibitor, the immunogenic cell death inducer and the immune checkpoint inhibitor according to embodiment 99, wherein the tumor is lung cancer, colon cancer (including colorectal cancer) or breast cancer.
101. A method for treating tumors by increasing immunogenic cell death in a subject, wherein the method comprises administering to the subject a therapeutically effective amount of an FAK inhibitor, an immunogenic cell death inducer and an immune checkpoint inhibitor, wherein the immunogenic cell death inducer is an RNA polymerase II inhibitor, an ALK/ROS1 inhibitor, a KRAS G12C inhibitor or a KRAS G12D inhibitor.
102. The method according to embodiment 101, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, and yet alternatively, the FAK inhibitor is IN10018, or a pharmaceutically acceptable salt thereof, alternatively, the FAK inhibitor is IN10018 tartrate, wherein the IN10018 has a structure of: 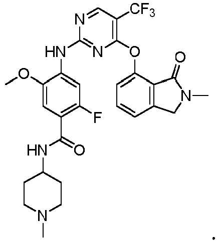
103. The method according to any one of embodiments 101-102, wherein the immunogenic cell death inducer is an RNA polymerase II inhibitor.
104. The method according to embodiment 103, wherein the RNA polymerase II inhibitor is Lurbinectedin, SEL-120, or a pharmaceutically acceptable salt thereof.
105. The method according to any one of embodiments 103-104, wherein the RNA polymerase II inhibitor is Lurbinectedin.
106. The method according to any one of embodiments 101-102, wherein the immunogenic cell death inducer is an ALK/ROS1 inhibitor.
107. The method according to embodiment 106, wherein the ALK/ROS1 inhibitor is Crizotinib, SIM-0201, XZP-3621, TQ-B3139, SAF-189s, Ceritinib, Lorlatinib (PF-06463922), Alectinib, Ensartinib, APG-2449, Brigatinib, TQ-B3101, Entrectinib, Repotrectinib, or a pharmaceutically acceptable salt thereof, alternatively, the ALK/ROS1 inhibitor is Crizotinib, Entrectinib, or a pharmaceutically acceptable salt thereof.
108. The method according to any one of embodiments 106-107, wherein the ALK/ROS1 inhibitor is Crizotinib, or a pharmaceutically acceptable salt thereof.
109. The method according to any one of embodiments 101-102, wherein the immunogenic cell death inducer is a KRAS G12C inhibitor.
110. The method according to embodiment 109, wherein the KRAS G12C inhibitor is D-1553, ARS-3248, GF-105, JAB-21822, JDQ-443, LY-3537982, Sotorasib (AMG510), Adagrasib (MRTX849), GDC-6036, or a pharmaceutically acceptable salt thereof, alternatively, the KRAS G12C inhibitor is D-1553, Sotorasib (AMG510), or a pharmaceutically acceptable salt thereof.
111. The method according to any one of embodiments 109-110, wherein the KRAS G12C inhibitor is D-1553, or a pharmaceutically acceptable salt thereof.
112. The method according to any one of embodiments 101-102, wherein the immunogenic cell death inducer is a KRAS G12D inhibitor.
113. The method according to embodiment 112, wherein the KRAS G12D inhibitor is MRTX1133, HRS-4642, JAB-22000, or a pharmaceutically acceptable salt thereof.
114. The method according to any one of embodiments 112-113, wherein the KRAS G12D inhibitor is MRTX1133, or a pharmaceutically acceptable salt thereof.
115. The method according to any one of embodiments 101-114, wherein the immune checkpoint inhibitor is an anti-PD-1/PD-L1 antibody, a PD-1/PD-L1 small molecule inhibitor, or a TIGIT antibody.
116. The method according to embodiment 115, wherein the immune checkpoint inhibitor is an anti-PD-1/PD-L1 antibody, alternatively, the anti-PD-1/PD-L1 antibody is pembrolizumab, tislelizumab, nivolumab, toripalimab, atezolizumab, durvalumab, avelumab, atezolizumab, camrelizumab, sintilimab, cemiplimab, envafolimab, BMS-936559, JS003, SHR-1316, GS-4224, AN-4005, or MX-10181.
117. The method according to embodiment 115, wherein the immune checkpoint inhibitor is a PD-1/PD-L1 small molecule inhibitor, alternatively, the PD-1/PD-L1 small molecule inhibitor is INCB-086550, Lazertinib, IMMH-010, CA-170, ABSK043, or RRx-001.
118. The method according to embodiment 115, wherein the immune checkpoint inhibitor is a TIGIT antibody, alternatively, the TIGIT antibody is ociperlimab (BGB-A1217), vibostolimab, domvanalimab (AB154), tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
119. The method according to embodiment 101, wherein the FAK inhibitor is IN10018 or a pharmaceutically acceptable salt thereof, the immunogenic cell death inducer is Lurbinectedin; and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.
120. The method according to embodiment 101, wherein the FAK inhibitor is IN10018 or a pharmaceutically acceptable salt thereof, the immunogenic cell death inducer is Crizotinib or a pharmaceutically acceptable salt thereof, and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.
121. The method according to embodiment 101, wherein the FAK inhibitor is IN10018 or a pharmaceutically acceptable salt thereof, the immunogenic cell death inducer is D-1553 or a pharmaceutically acceptable salt thereof, and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.
122. The method according to embodiment 101, wherein the FAK inhibitor is IN10018 or a pharmaceutically acceptable salt thereof, the immunogenic cell death inducer is MRTX1133 or a pharmaceutically acceptable salt thereof, and the immune checkpoint inhibitor is a TIGIT antibody.
123. The method according to any one of embodiments 101-122, wherein the FAK inhibitor, the immunogenic cell death inducer, and the immune checkpoint inhibitor are administered simultaneously or sequentially to the subject.
124. The method according to any one of embodiments 101-123, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
125. The method according to embodiment 124, wherein the tumor is lung cancer, colon cancer (including colorectal cancer) or breast cancer.
126. Use of an FAK inhibitor in the manufacture of a medicament for treating tumors in a subject, wherein the FAK inhibitor, an immunogenic cell death inducer and an immune checkpoint inhibitor are administered to the subject, wherein the immunogenic cell death inducer is an RNA polymerase II inhibitor, an ALK/ROS1 inhibitor, a KRAS G12C inhibitor or a KRAS G12D inhibitor.
127. Use of an immunogenic cell death inducer in the manufacture of a medicament for treating tumors in a subject, wherein an FAK inhibitor, the immunogenic cell death inducer and an immune checkpoint inhibitor are administered to the subject, wherein the immunogenic cell death inducer is an RNA polymerase II inhibitor, an ALK/ROS1 inhibitor, a KRAS G12C inhibitor or a KRAS G12D inhibitor.
128. Use of an immune checkpoint inhibitor in the manufacture of a medicament for treating tumors in a subject, wherein an FAK inhibitor, an immunogenic cell death inducer, and the immune checkpoint inhibitor are administered to the subject, wherein the immunogenic cell death inducer is an RNA polymerase II inhibitor, an ALK/ROS1 inhibitor, a KRAS G12C inhibitor or a KRAS G12D inhibitor.
129. Use of an FAK inhibitor, an immunogenic cell death inducer and an immune checkpoint inhibitor in the manufacture of a combined medicament for treating tumors, wherein the immunogenic cell death inducer is an RNA polymerase II inhibitor, an ALK/ROS1 inhibitor, a KRAS G12C inhibitor or a KRAS G12D inhibitor.
130. Use of an FAK inhibitor in the manufacture of a combined medicament with an immunogenic cell death inducer and an immune checkpoint inhibitor for treating tumors, wherein the immunogenic cell death inducer is an RNA polymerase II inhibitor, an ALK/ROS1 inhibitor, a KRAS G12C inhibitor or a KRAS G12D inhibitor.
131. Use of an immunogenic cell death inducer in the manufacture of a combined medicament with an FAK inhibitor and an immune checkpoint inhibitor for treating tumors, wherein the immunogenic cell death inducer is an RNA polymerase II inhibitor, an ALK/ROS1 inhibitor, a KRAS G12C inhibitor or a KRAS G12D inhibitor.
132. Use of an immune checkpoint inhibitor in the manufacture of a combined medicament with an FAK inhibitor and an immunogenic cell death inducer for treating tumors, wherein the immunogenic cell death inducer is an RNA polymerase II inhibitor, an ALK/ROS1 inhibitor, a KRAS G12C inhibitor or a KRAS G12D inhibitor.
133. Use of an FAK inhibitor, an immunogenic cell death inducer and an immune checkpoint inhibitor in the manufacture of a medicament for the combined treatment of tumors, wherein the immunogenic cell death inducer is an RNA polymerase II inhibitor, an ALK/ROS1 inhibitor, a KRAS G12C inhibitor or a KRAS G12D inhibitor.
134. Use of an FAK inhibitor in the manufacture of a medicament for the combined treatment of tumors with an immunogenic cell death inducer and an immune checkpoint inhibitor, wherein the immunogenic cell death inducer is an RNA polymerase II inhibitor, an ALK/ROS1 inhibitor, a KRAS G12C inhibitor or a KRAS G12D inhibitor.
135. Use of an immunogenic cell death inducer in the manufacture of a medicament for the combined treatment of tumors with an FAK inhibitor and an immune checkpoint inhibitor, wherein the immunogenic cell death inducer is an RNA polymerase II inhibitor, an ALK/ROS1 inhibitor, a KRAS G12C inhibitor or a KRAS G12D inhibitor.
136. Use of an immune checkpoint inhibitor in the manufacture of a medicament for the combined treatment of tumors with an FAK inhibitor and an immunogenic cell death inducer, wherein the immunogenic cell death inducer is an RNA polymerase II inhibitor, an ALK/ROS1 inhibitor, a KRAS G12C inhibitor or a KRAS G12D inhibitor.
137. The use according to any one of embodiments 126-136, wherein the FAK inhibitor is IN10018, Defactinib, GSK2256098, PF-00562271, VS-4718, APG-2449, AMP945, AMP886 or a pharmaceutically acceptable salt thereof, alternatively IN10018, Defactinib, AMP945 or a pharmaceutically acceptable salt thereof, yet alternatively IN10018 or a pharmaceutically acceptable salt thereof, alternatively IN10018 tartrate, wherein the structure of IN10018 is: 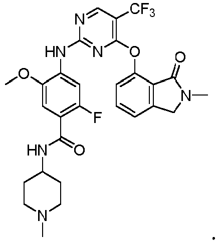
138. The use according to any one of embodiments 126-137, wherein the immunogenic cell death inducer is an RNA polymerase II inhibitor.
139. The use according to embodiment 138, wherein the RNA polymerase II inhibitor is Lurbinectedin, SEL-120 or a pharmaceutically acceptable salt thereof.
140. The use according to any one of embodiments 138-139, wherein the RNA polymerase II inhibitor is Lurbinectedin.
141. The use according to any one of embodiments 126-137, wherein the immunogenic cell death inducer is an ALK/ROS1 inhibitor.
142. The use according to embodiment 141, wherein the ALK/ROS1 inhibitor is Crizotinib, SIM-0201, XZP-3621, TQ-B3139, SAF-189s, Ceritinib, Lorlatinib (PF-06463922), Alectinib, Ensartinib, APG-2449, Brigatinib, TQ-B3101, Entrectinib, Repotrectinib or a pharmaceutically acceptable salt thereof, alternatively Crizotinib, Entrectinib or a pharmaceutically acceptable salt thereof.
143. The use according to any one of embodiments 141-142, wherein the ALK/ROS1 inhibitor is Crizotinib or a pharmaceutically acceptable salt thereof.
144. The use according to any one of embodiments 126-137, wherein the immunogenic cell death inducer is a KRAS G12C inhibitor.
145. The use according to embodiment 144, wherein the KRAS G12C inhibitor is D-1553, ARS-3248, GF-105, JAB-21822, JDQ-443, LY-3537982, Sotorasib (AMG510), Adagrasib (MRTX849), GDC-6036 or a pharmaceutically acceptable salt thereof, alternatively D-1553, Sotorasib (AMG510) or a pharmaceutically acceptable salt thereof.
146. The use according to any one of embodiments 144-145, wherein the KRAS G12C inhibitor is D-1553 or a pharmaceutically acceptable salt thereof.
147. The use according to any one of embodiments 126-137, wherein the immunogenic cell death inducer is a KRAS G12D inhibitor.
148. The use according to embodiment 147, wherein the KRAS G12D inhibitor is MRTX1133, HRS-4642, JAB-22000 or a pharmaceutically acceptable salt thereof.
149. The use according to any one of embodiments 147-148, wherein the KRAS G12D inhibitor is MRTX1133 or a pharmaceutically acceptable salt thereof.
150. The use according to any one of embodiments 126-149, wherein the immune checkpoint inhibitor is an anti-PD-1/PD-L1 antibody, a PD-1/PD-L1 small molecule inhibitor or a TIGIT antibody.
151. The use according to embodiment 150, wherein the immune checkpoint inhibitor is an anti-PD-1/PD-L1 antibody, and alternatively the anti-PD-1/PD-L1 antibody is pembrolizumab, Tislelizumab, Nivolumab, Toripalimab, Atezolizumab, durvalumab, Avelumab, Atezolizumab, Camrelizumab, Sintilimab, Cemiplimab, envafolimab, BMS-936559, JS003, SHR-1316, GS-4224, AN-4005, or MX-10181.
152. The use according to embodiment 150, wherein the immune checkpoint inhibitor is a PD-1/PD-L1 small molecule inhibitor, and alternatively the PD-1/PD-L1 small molecule inhibitor is INCB-086550, Lazertinib, IMMH-010, CA-170, ABSK043 or RRx-001.
153. The use according to embodiment 150, wherein the immune checkpoint inhibitor is a TIGIT antibody, and alternatively, the TIGIT antibody is Ociperlimab (BGB-A1217), Vibostolimab, domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
154. The use according to any one of embodiments 126-136, wherein the FAK inhibitor is IN10018 or a pharmaceutically acceptable salt thereof, the immunogenic cell death inducer is Lurbinectedin, and the immune checkpoint inhibitor is an anti-PD-1/PD-L1 antibody or a PD-1/PD-L1 small molecule inhibitor, and alternatively, the immune checkpoint inhibitor is an anti-PD-1/PD-L1 antibody.
155. The use according to any one of embodiments 126-136, wherein the FAK inhibitor is IN10018 or a pharmaceutically acceptable salt thereof, the immunogenic cell death inducer is Crizotinib or a pharmaceutically acceptable salt thereof, and the immune checkpoint inhibitor is an anti-PD-1/PD-L1 antibody or a PD-1/PD-L1 small molecule inhibitor, and alternatively, the immune checkpoint inhibitor is an anti-PD-1/PD-L1 antibody.
156. The use according to any one of embodiments 126-136, wherein the FAK inhibitor is IN10018 or a pharmaceutically acceptable salt thereof, the immunogenic cell death inducer is D-1553 or a pharmaceutically acceptable salt thereof, and the immune checkpoint inhibitor is an anti-PD-1/PD-L1 antibody or a PD-1/PD-L1 small molecule inhibitor, and alternatively, the immune checkpoint inhibitor is an anti-PD-1/PD-L1 antibody.
157. The use according to any one of embodiments 126-136, wherein the FAK inhibitor is IN10018 or a pharmaceutically acceptable salt thereof, the immunogenic cell death inducer is MRTX1133 or a pharmaceutically acceptable salt thereof, and the immune checkpoint inhibitor is a TIGIT antibody.
158. The use according to any one of embodiments 126-157, wherein the FAK inhibitor, the immunogenic cell death inducer and the immune checkpoint inhibitor are administered simultaneously or sequentially to the subject.
159. The use according to any one of embodiments 126-158, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
160. The use according to embodiment 159, wherein the tumor is lung cancer, colon cancer (including colorectal cancer) or breast cancer.

### Examples

The following examples are provided to further illustrate the present disclosure. It should be understood that these examples are only used to illustrate the present disclosure and are not used to limit the scope of the present disclosure.

The assay methods without specific conditions in the following examples can be carried out according to the conventional conditions of this kind of reaction or according to the conditions suggested by the manufacturer. The assay materials and reagents used in the following examples are all commercially available unless otherwise specified.

The abbreviations used in the examples have the following meanings:

| Abbreviation | Name |
|---|---|
| siRNA | Small interfering RNA |
| DMSO | Dimethyl sulfoxide |
| COMBO | Combination |
| HMGB1 | High mobility group box-1 protein |
| AAALAC | Association for Assessment and Accreditation of Laboratory Animal Care |
| IACUC | Institutional Animal Care and Use Committee |
| SPF | Specific pathogen Free |
| BIW | Twice a week |
| BID | Twice a day |
| QD | Once a day |
| BW | Body weight |
| EDTA | Ethylenediamine tetraacetic acid |
| GLP | Good Laboratory Practice for Non-clinical Laboratory Studies |
| *p.o.* | Oral gavage |
| *i.v.* | Intravenous administration |
| *i.p.* | Intraperitoneal injection administration |
| DPBS | Dulbecco's Phosphate Buffered Saline |
| DDH2O | Double distilled water |
| RT | Room temperature |
| SEM | Standard error of the mean |
| TGI | Tumor growth inhibition rate |
| TV | Tumor volume |
| TW | Tumor weight |
| FBS | Fetal bovine serum |
| PBS | Phosphate-buffered saline |
| MC | Methylcellulose |
| CO₂ | Carbon dioxide |
| CRT | Calreticulin |
| Annexin-V-FITC | Fluorescein isothiocyanate-labeled annexin |
| PI | Propidium iodide |

### Example 1: Synergistic effects of IN10018 and Lurbinectedin in Colon Cancer CT26 Cells

Mouse colon cancer CT26 cells (Insitute of Biochemistry and Cell Biology) were cultured with RPMI-1640 (Shanghai Basalmedia, classification number: L210KJ, batch number: F210916)+10%FBS (Gibco, classification number: 10099-141c, batch number: 2158737cp) and passaged twice. When the cells were in good condition, the culture solution was placed in a 96-well plate with 3000 cells/well. After the cells were spread for 24 hours, a culture medium containing Lurbinectedin (DC chemicals, classification number: DC12502, batch number: DC1250203) was added. 10 drug action concentrations were set, wherein the first concentration was 0.1 µM, which was serially diluted 4 times, and the last concentration was a control with a drug concentration of 0. Each drug concentration was set in triplicate. At the same time, another group of cells was established with the same drug concentration as the above method. The difference was that 6µM IN10018 was added to each well. The drugs were mixed and the mixture was cultured in a 5%CO₂ incubator at 37°C for 72 hours.

After 72 hours of drug action, the cells were observed under a microscope, and 10 µl of CCK8 detection reagent (Cellorlab, classification number: CX001M, batch number: 2571100) was added to each well. The mixture was cultured in a 5%CO₂ incubator at 37°C for 2-4 hours. Then the plate was read with a microplate reader OD450 (chemiluminescence method).

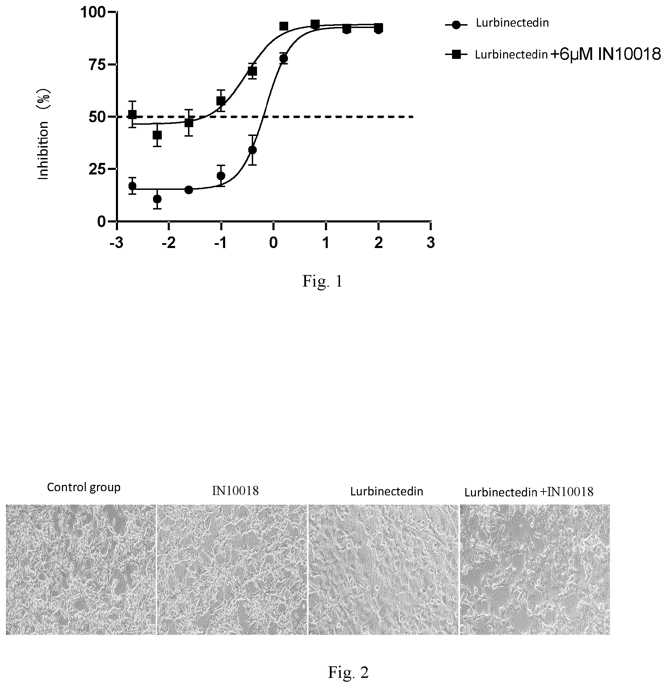

The results showed that the IC50 of the Lurbinectedin group was 0.71nM; and the IC50 of the Lurbinectedin+6µM IN10018 group was 0.30nM. The IC50 of the group containing IN10018 is lower than that of the group without IN10018, indicating that the therapeutic effect of the combined treatment group of two drugs was better than that of the single drug treatment group, as shown in Figure 1.

### Example 2: Study of IN10018 and Lurbinectedin in Colon Cancer CT26 Cells

Mouse colon cancer CT26 cells (Insitute of Biochemistry and Cell Biology) were cultured with RPMI-1640 (Shanghai Basalmedia, classification number: L210KJ, batch number: F210916)+10%FBS (Gibco, classification number: 10099-141c, batch number: 2158737cp) and passaged twice. When the cells were in good condition, the culture solution was placed in a 24-well plate. After the cells were spread for 24 hours, four groups were set up, wherein the first group was a control group, and the culture medium was added; the second group was IN10018 with a concentration of 5µM; the third group was Lurbinectedin (DC chemicals, classification number: DC12502, batch number: DC1250203) with a concentration of 0.00142µM; and the fourth group was IN10018 (5µM) combined with Lurbinectedin (0.00142µM). The drugs were mixed and the mixture was cultured in a 5%CO₂ incubator at 37°C for 48 hours.

After 48 hours of drug action, the cells were observed under a microscope and photographed, and the photos were kept. Then the cells were collected, subjected to flow analysis, and washed twice with flow buffer (PBS+2%FBS). 0.5 µl of AF647 anti-Calreticulin antibody (abcam, classification number: ab196159, batch number: CR33676773) was added to each well and fully mixed. The mixture was incubated at 4°C for 20 minutes in the dark. After 20 minutes, the cells were washed twice with flow buffer (PBS+2%FBS). To apoptosis detection kit (Beyotime, catalog number: CL062L, batch number: 021921210811) was added 195µl of annexin-V-FITC conjugate. The cells were gently mixed. 5µl of annexin-V-FITC antibody was added, and gently mixed. 10µl of PI dye was added, and gently mixed. The mixture was cultured at room temperature in the dark for 15 minutes, and then analyzed by flow cytometry.

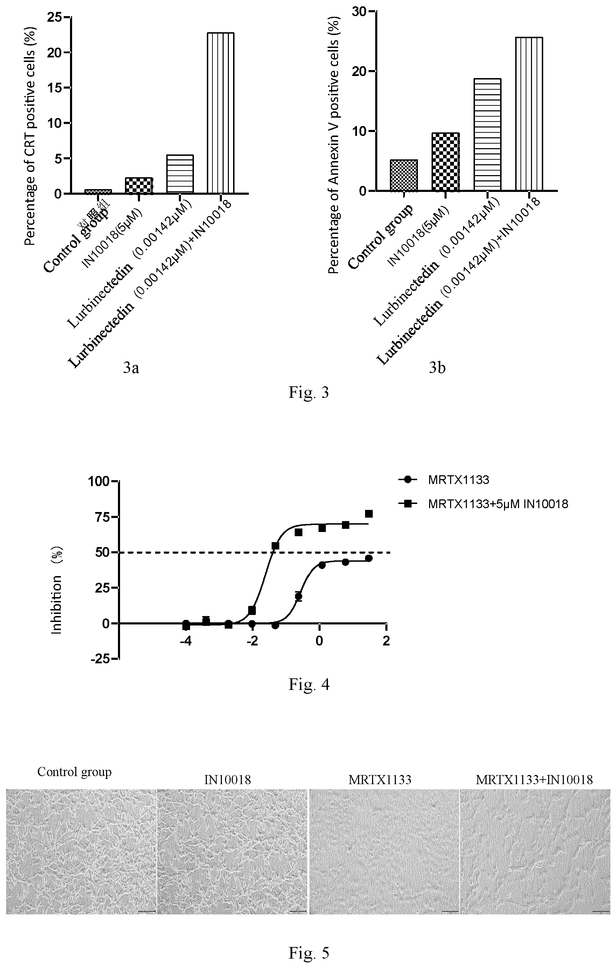

The cells were observed under a microscope. The cell state of the Lurbinectedin single-drug group and two-drug combination group was poor, wherein the two-drug combination group had the worst cell state with more cell deaths. The cell state of the control group and IN10018 group was good, as shown in Figure 2. The results showed that the CRT positive rate and Annexin V positive rate in the two-drug combination group were higher than those in the single drug group, as shown in Figure 3.

### Example 3: Synergistic effect of IN10018 and MRTX1133 in colon cancer CT26 cells

Mouse colon cancer CT26 cells (Insitute of Biochemistry and Cell Biology) were cultured with RPMI-1640 (Shanghai Basalmedia, classification number: L210KJ, batch number: F210916)+10%FBS (Gibco, classification number: 10099-141c, batch number: 2158737cp) and passaged twice. When the cells were in good condition, the culture solution was placed in a 96-well plate with 3000 cells/well. After the cells were spread for 24 hours, a culture medium containing MRTX1133 (SuperLan Chemical Co., Ltd., classification number: P2621928-55-8, batch number: 131005) was added. 10 drug action concentrations were set, wherein the first concentration was 30 µM, which was serially diluted 5 times, and the last concentration was a control with a drug concentration of 0. Each drug concentration was set in triplicate. At the same time, another group of cells was established with the same drug concentration as the above method. The difference was that 5µM IN10018 was added to each well. The drugs were mixed and the mixture was cultured in a 5%CO₂ incubator at 37°C for 72 hours.

After 72 hours of drug action, the cells were observed under a microscope, and 10 µl of CCK8 detection reagent (Cellorlab, classification number: CX001M, batch number: 2571100) was added to each well. The mixture was cultured in a 5%CO₂ incubator at 37°C for 2-4 hours. Then the plate was read with a microplate reader OD450 (chemiluminescence method).

The results showed that the IC50 of the MRTX1133 group was 0.28µM; and the IC50 of the MRTX1133+5µM IN10018 group was 0.02µM. The IC50 of the group containing IN10018 was significantly lower than that of the group without IN10018, indicating that the therapeutic effect of the combined treatment group of two drugs was better than that of the single drug treatment group, as shown in Figure 4.

### Example 4: Study of IN10018 and MRTX1133 in Colon Cancer CT26 Cells

Mouse colon cancer CT26 cells (Insitute of Biochemistry and Cell Biology) were cultured with RPMI-1640 (Shanghai Basalmedia, classification number: L210KJ, batch number: F210916)+10%FBS (Gibco, classification number: 10099-141c, batch number: 2158737cp) and passaged twice. When the cells were in good condition, the culture solution was placed in a 24-well plate. After the cells were spread for 24 hours, four groups were set up, wherein the first group was a control group, and the culture medium was added; the second group was IN10018 with a concentration of 5µM; the third group was MRTX1133 (SuperLan Chemical Co., Ltd., classification number: P2621928-55-8, batch number: 131005) with a concentration of 6 µM; and the fourth group was IN10018 (5µM) combined with MRTX1133 (6 µM). The drugs were mixed and the mixture was cultured in a 5%CO₂ incubator at 37°C for 48 hours.

After 48 hours of drug action, the cells were observed under a microscope and photographed, and the photos were kept. Then the cells were collected, subjected to flow analysis, and washed twice with flow buffer (PBS+2%FBS). 0.5 µl of AF647 anti-Calreticulin antibody (abcam, classification number: ab196159, batch number: CR33676773) was added to each well and fully mixed. The mixture was incubated at 4°C for 20 minutes in the dark. After 20 minutes, the cells were washed twice with flow buffer (PBS+2%FBS). To apoptosis detection kit (Beyotime, catalog number: CL062L, batch number: 021921210811) was added 195µl of annexin-V-FITC conjugate. The cells were gently mixed. 5µl of annexin-V-FITC antibody was added, and gently mixed. 10µl of PI dye was added and mixed. The mixture was cultured at room temperature in the dark for 15 minutes, and then analyzed by flow cytometry.

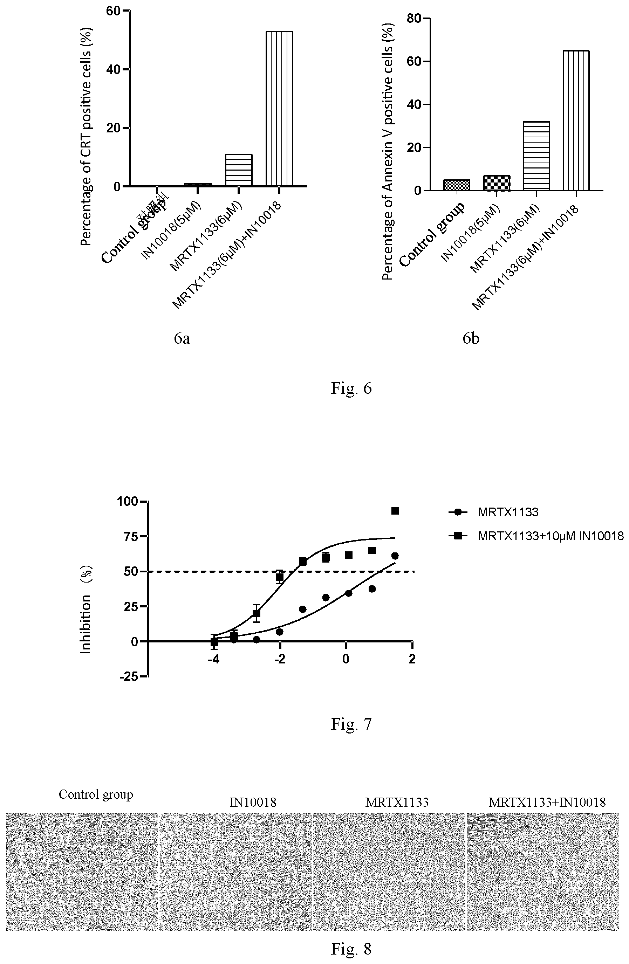

The cells were observed under a microscope. The cell state of the MRTX1133 single-drug group and two-drug combination group was poor, wherein the two-drug combination group had the worst cell state with more cell deaths. The cell state of the control group and IN10018 group was good, as shown in Figure 5. The results showed that the CRT positive rate and Annexin V positive rate in the two-drug combination group were higher than those in the single drug group, as shown in Figure 6.

### Example 5: Synergistic effect of IN10018 and MRTX1133 in lung cancer KPL cells

Mouse lung cancer KPL (Insitute of Biochemistry and Cell Biology) were cultured with RPMI-1640 (Shanghai Basalmedia, classification number: L210KJ, batch number: F210916)+10%FBS (Gibco, classification number: 10099-141c, batch number: 2158737cp) and passaged twice. When the cells were in good condition, the culture solution was placed in a 96-well plate with 3000 cells/well. After the cells were spread for 24 hours, a culture medium containing MRTX1133 (SuperLan Chemical Co., Ltd., classification number: P2621928-55-8, batch number: 131005) was added. 10 drug action concentrations were set, wherein the first concentration was 30 µM, which was serially diluted 5 times, and the last concentration was a control with a drug concentration of 0. Each drug concentration was set in triplicate. At the same time, another group of cells was established with the same drug concentration as the above method. The difference was that 10µM IN10018 was added to each well. The drugs were mixed and the mixture was cultured in a 5%CO₂ incubator at 37°C for 72 hours.

After 72 hours of drug action, the cells were observed under a microscope, and 10 µl of CCK8 detection reagent (Cellorlab, classification number: CX001M, batch number: 2571100) was added to each well. The mixture was cultured in a 5%CO₂ incubator at 37°C for 2-4 hours. Then the plate was read with a microplate reader OD450 (chemiluminescence method).

The results showed that the IC50 of the MRTX1133 group was 1.76 µm; and the IC50 of the MRTX1133+10µM IN10018 group was 0.008µM. The IC50 of the group containing IN10018 was significantly lower than that of the group without IN10018, indicating that the therapeutic effect of the combined treatment group of two drugs was better than that of the single drug treatment group, as shown in Figure 7.

### Example 6: Study of IN10018 and MRTX1133 in lung cancer KPL cells

Mouse lung cancer KPL (Insitute of Biochemistry and Cell Biology) were cultured with RPMI-1640 (Shanghai Basalmedia, classification number: L210KJ, batch number: F210916)+10%FBS (Gibco, classification number: 10099-141c, batch number: 2158737cp) and passaged twice. When the cells were in good condition, the culture solution was placed in a 24-well plate. After the cells were spread for 24 hours, four groups were set up, wherein the first group was a control group, and the culture medium was added; the second group was IN10018 with a concentration of 10µM; the third group was MRTX1133 (SuperLan Chemical Co., Ltd., classification number: P2621928-55-8, batch number: 131005) with a concentration of 15 µM; and the fourth group was IN10018 (10 µM) combined with MRTX1133 (15 µM). The drugs were mixed and the mixture was cultured in a 5%CO₂ incubator at 37°C for 48 hours.

After 48 hours of drug action, the cells were observed under a microscope and photographed, and the photos were kept. Then the cells were collected, subjected to flow analysis, and washed twice with flow buffer (PBS+2%FBS). 0.5 µl of AF647 anti-Calreticulin antibody (abcam, classification number: ab196159, batch number: CR33676773) was added to each well and fully mixed. The mixture was incubated at 4°C for 20 minutes in the dark. After 20 minutes, the cells were washed twice with flow buffer (PBS+2%FBS). To apoptosis detection kit (Beyotime, catalog number: CL062L, batch number: 021921210811) was added 195µl of annexin-V-FITC conjugate. The cells were gently mixed. 5µl of annexin-V-FITC antibody was added, and gently mixed. 10µl of PI dye was added, and mixed. The mixture was cultured at room temperature in the dark for 15 minutes, and then analyzed by flow cytometry.

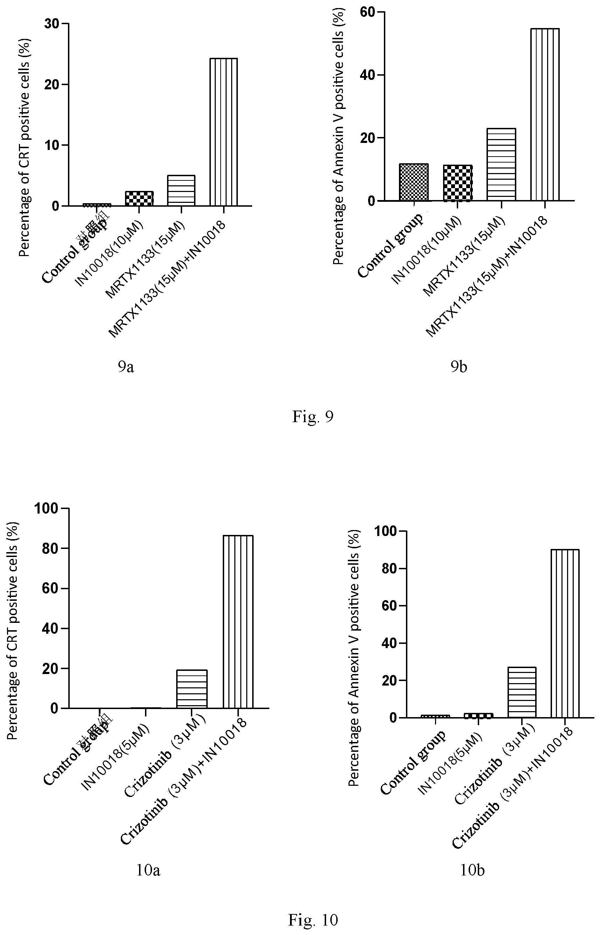

The cells were observed under a microscope. The cell state of the MRTX1133 single-drug group and two-drug combination group was poor, wherein the two-drug combination group had the worst cell state with more cell deaths. The cell state of the control group and IN10018 group was good, as shown in Figure 8. The results showed that the CRT positive rate and Annexin V positive rate in the two-drug combination group were higher than those in the single drug group, as shown in Figure 9.

### Example 7: Study of the Induction of Immunogenic Cell Death Targets by Crizotinib and IN10018 in Mouse Colon Cancer CT26 Cells

The compound information was shown in Table 1:

**Table 1.**

| Drug name | Storage condition | Source | Solvent |
|---|---|---|---|
| Crizotinib | 4°C | MCE | DMSO |
| IN10018 | 4°C | Synthesized according to the method in patent WO2010058032 | DMSO |

The main reagent information of the assay was shown in Table 2:

**Table 2.**

| Reagent Name | Manufacturer | Article number | Batch number |
|---|---|---|---|
| DPBS | Shanghai Basalmedia | B210KJ | B210902 |
| RPMI-1640 | Shanghai Basalmedia | L210KJ | F210916 |
| FBS | GIBCO | 10099-141c | 2158737cp |
| Trypsin | Shanghai Basalmedia | S310KJ | C210903 |
| Annexin V-apoptosis detection kit | Beyotime | C1062L | 021921210811 |
| AF647 Anti-Calreticulin Antibody | Abcam | ab196159 | CR33676773 |

The assay design was shown in Table 3:

**Table 3.**

| No. | Drug |
|---|---|
| 1 | Control group |
| 2 | IN10018 (5µM) |
| 3 | Crizotinib (3µM) |
| 4 | Crizotinib (3µM)+IN10018 (5µM) |

### Cell culture:

CT26 cells were cultured in monolayer in vitro, and the culture conditions were: RPMI-1640 medium with 10% fetal bovine serum, 37 °C and 5% CO₂. Trypsin was used for routine digestion and passage treatment two or three times a week. When the cells were in exponential growth period and the adherent confluence reached 80%-90%, the cells were collected and plated.

CT26 cells were digested with trypsin, then collected and counted. According to the counting results, the cells were diluted with RPMI-1640+10%FBS, and the dilution concentration was 50,000 cells per ml. Then, the cells were plated on 24-well culture plates, and 1 ml of cell suspension (50,000 cells) was laid in each well. After plating, the cells were cultured in 37 °C and 5% CO₂ incubator.

Adding the compound to be assayed:
After 24 hours of plating, the compounds to be assayed, IN10018 and crizotinib, were added into different wells, and the grouping and drug concentration were shown in Table 3.

Collecting cells for flow detection:
After 48 hours of drug action, the cells were photographed by microscope, digested by trypsin and collected for flow staining.

After the cells were washed twice with flow buffer (DPBS+2% FBS), the cells in each group were divided into two parts on average, one of which was added with 0.5 µl of AF647 anti-Calreticulin antibody in each well, then mixed well, and incubated in the dark for 20 minutes at 4°C. After that, the wells were washed once with flow buffer. Then 195 µl of Annexin-V-FITC binding solution was added into the wells, and the cells were gently mixed. 5 µl of Annexin-V-FITC antibody was added to the wells, and further mixed well. Then 10 µl of PI dye was added, and mixed well. The cells were cultured at room temperature in the dark for 15 minutes, and then analyzed by flow cytometry.

Data analysis: After the assay, the cell positive rate was analyzed by Flowjo(V10) software.

### Assay results:

After 48 hours of drug action, the cell state of each group was observed under a microscope. The cell death was obvious in the single drug group of crizotinib, and the cell death was the most obvious in the combined drug group. The cell viability of the control group and IN10018 group was better. The results of flow cytometry showed that the CRT and Annexin-V positive rates in the combined drug group were significantly higher than those in the single drug group. The related detection results were shown in Figure 10.

### Example 8: In vivo pharmacodynamic study of the assay drug in BALB/c mouse subcutaneous allograft tumor model of mouse colon cancer CT26-KRAS G12C cells.

### Assay materials:

Species: mice; Strain: BALB/c mice; Age and weight: 6-8 weeks old, weighing: 18-22g; Gender: female; Quantity: 49 (excluding remaining mice after grouping); Supplier: LINGCHANG BIOTECH.

After the animals arrived, they were raised in the assay environment for 3-7 days before the assay. Animals were raised in cages with IVC (independent air supply system) in SPF animal rooms (3~4 animals per cage). The animal information card for each cage indicated the number, sex, strain, receiving date, administration plan, assay number, group and assay start date of the animals in the cage. All cages, padding and drinking water were sterilized before use. Cages, feed and drinking water should be replaced twice a week. The feeding environment and illumination condition were as follows: temperature: 20 ~ 26 °C; Humidity: 40 ~ 70%; Lighting period: 12 hours of light, 12 hours of no light (turning on the light at 8: 00 am to turning off the light at 8: 00 pm); Cage: made of polycarbonate, with a volume of 300 mm x 180 mm x 150 mm. The padding was corncob, which was replaced twice a week; Food: assay animals can eat freely during the whole assay (radiation sterilization, dry granular food); Drinking water: assay animals can drink freely sterilized water; Animal identification: assay animals were identified by ear tags.

The information of the assay sample and control sample was shown in Table 4.

**Table 4**

| Drug name | Storage condition | Source |
|---|---|---|
| IN10018 | 4°C | Synthesized according to the method in patent WO2010058032. |
| D-1553 | 4°C | InventisBio |
| INX0082 | 4°C | Bioxcell |

| | | |
|---|---|---|
| Note: (INX0082 is PD1 antibody). | | |

### Assay methods and steps:

Mouse colon cancer CT26-KRAS G12C cells were cultured in monolayer in vitro. The culture conditions were RPMI1640 medium with 10% fetal bovine serum, 1% penicillin/streptomycin/amphotericin B, and 37°C 5%CO₂ incubator. Trypsin-EDTA was used twice a week for routine digestion and passage. When the cell saturation was 80%-90% and the number reached the requirements, the cells were collected, counted and inoculated.

0.1 mL (0.3×10⁶ cells) of CT26-KRAS G12C cells were subcutaneously inoculated on the right back of each mouse, and grouping and drug administration began when the average tumor volume reached about 55 mm³. The grouping information was shown in Table 5.

**Table 5**

| Group | N¹ | Compounds for treatment | Dose (mg/kg) | Dosage volume parameter (µL/g)² | Route of administration | Frequency of administration |
|---|---|---|---|---|---|---|
| 1 | 7 | Solvent control | -- | 10 | PO | QD X 28 days |
| 2 | 7 | D-1553 | 5 | 10 | PO | QD X 28 days |
| 3 | 7 | IN10018 | 25 | 10 | PO | QD X 28 days |
| 4 | 7 | D-1553+IN10018 | 5+25 | 10 | PO; PO | QD X 28 days; QD X 28 days |
| 5 | 7 | INX0082 | 10 | 10 | IP | BIW X 4 weeks |
| 6 | 7 | D-1553+INX0082 | 5+10 | 10 | PO; IP | QD X 28 days; BIW X 4 weeks |
| 7 | 7 | D-1553+IN10018+INX0082 | 5+25+10 | 10 | PO; PO; IP | QD X 28 days; QD X 28 days; BIW X 4 weeks |

| | | | | | | |
|---|---|---|---|---|---|---|
| Note: 1. N: number of mouse in each group; | | | | | | |

2. Dosage volume: 10 µl/g according to the body weight of mouse. If body weight loss exceeded 15%, the dosage regimen should be adjusted accordingly.

The preparation of the assay substance was shown in Table 6.

**Table 6: Preparation method of assay substance**

| Compound | Packaging | Preparation method | Concentration (mg/mL) | Storage condition |
|---|---|---|---|---|
| Solvent | -- | 10% DMAC + 10% Solutol HS 15 + 80% HPβCD (10% HPβCD aqueous solution) | -- | 4°C |
| D-1553 | 250.11mg | 59.62 mg of D-1553 was weighed, and 5.88 ml of DMAC was added. The mixture was vortexed and ultrasonicated to obtain a uniform suspension. To the suspension was added 5.88 ml of Solutol HS 15, and the mixture was vortexed and ultrasonicated to obtain a uniform suspension. Finally, to the suspension was added 47.04 mL of 10% HPβCD aqueous solution, and the mixture was vortexed and ultrasonicated to obtain a uniform suspension, which could be used for one week. | 1 mg/ml | 4°C |
| IN10018 | 601.84mg | 116.09 mg of IN10018 was weighed, and 44.1 ml of PBS was added. The mixture was vortexed and ultrasonicated to obtain a uniform suspension, which could be used for one week. | 2.5 mg/ml | 4°C |
| INX0082 | 9.28mg/mL | 0.679 mL of 9.28 mg/mL INX0082 was weighed into a dispensing bottle. To the bottle was added 5.622 ml of PBS for dilution to obtain a working solution with a concentration of 1 mg/ml. | 1 mg/ml | 4°C |

| | | | | |
|---|---|---|---|---|
| Note: It was necessary to gently and fully mix the drugs before administering to animals. | | | | |

### Daily observation of assay animals:

The health status and death of animals were monitored every day. Routine examination included observing the effects of tumor growth and drug treatment on the daily behavior of animals, such as behavioral activities, food and water intake (visual observation only), body weight change (body weight measurement three times a week), appearance signs or other abnormal conditions. Based on the number of animals in each group, the animal death number and side effect in the group were recorded.

### Tumor measurement and assay index:

The diameter of tumor was measured three times a week with vernier caliper. The formula for calculating the tumor volume is: V = 0.5× *a* × *b*², where *a* and *b* represent the long and short diameters of the tumor respectively.

The anti-tumor effect of the compound was evaluated by TGI (%) or relative tumor proliferation rate T/C(%). The relative tumor proliferation rate is T/C(%) = T_{RTV} / C_{RTV} × 100 % (T_{RTV}: RTV in treatment group; C_{RTV}: RTV in negative control group). According to the results of tumor measurement, the relative tumor volume (RTV) was calculated, and the calculation formula is RTV = Vₜ / V₀, where V₀ is the average tumor volume measured at the time of grouping and drug administration (i.e., D0), Vₜ is the average tumor volume measured at a certain time, and data on the same day were used for T_{RTV} and C_{RTV}.TGI (%) reflects the tumor growth inhibition rate. TGI (%) = [1 - (average tumor volume at the end of administration in a certain treatment group-average tumor volume at the beginning of administration in the treatment group)/(average tumor volume at the end of administration in solvent control group-average tumor volume at the beginning of administration in the solvent control group)] × 100%.

### Statistical analysis:

Statistical analysis included the mean and standard error (SEM) of tumor volume at each time point in each group (see the specific data in Table 7). Based on the tumor volume data on the 19th day after administration, statistical analysis was performed to evaluate the differences between groups. T-test was used to analyze the comparison between two groups, and one-way ANOVA was used to analyze the comparison between three or more groups. If there was significant difference in F value, Games-Howell method was used for the test. If there was no significant difference in F value, Dunnet (2-sided) method was used for analysis. All data were analyzed by SPSS 17.0. P < 0.05 was considered to have significant difference.

### Assay results:

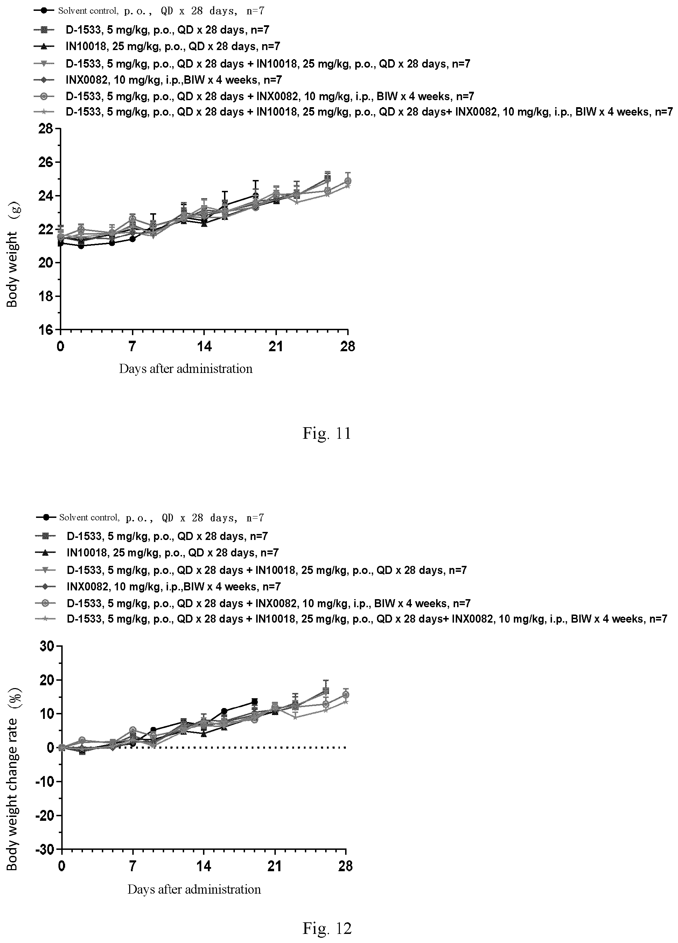

The body weight of assay animals was used as a reference index for indirect determination of drug toxicity. In this model, all animals in the administration groups did not show significant weight loss (as shown in Figure 11), and there was no morbidity or death in mice.

The effect of the assay substance on the body weight of female BALB/c mouse subcutaneous allograft tumor model of mouse colon cancer CT26-KRAS G12C cells was shown in Figures 11 and 12.

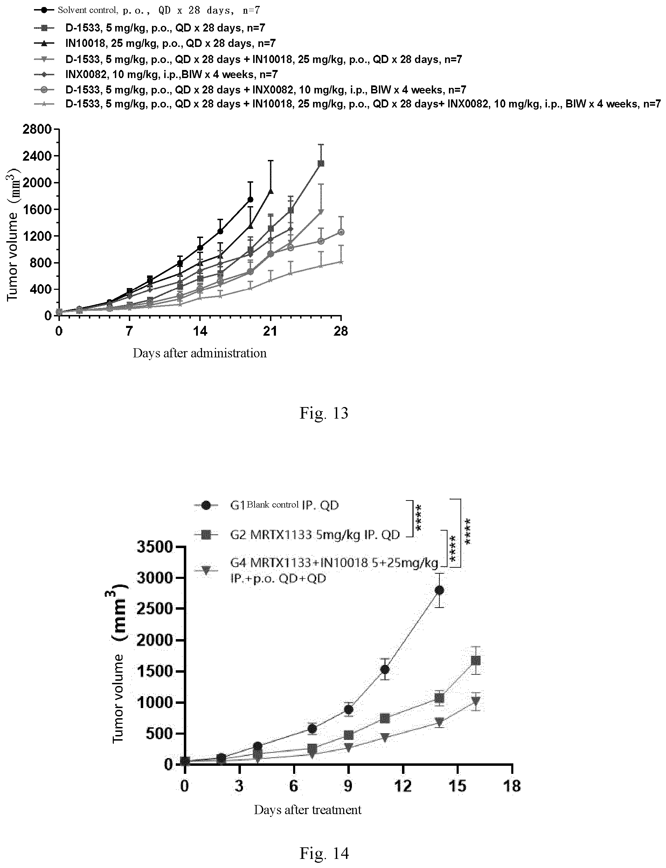

In this assay, in vivo efficacy of single and combined administration of the assay substance in mouse colon cancer CT26-KRAS G12C allograft tumor model was evaluated. On the 19th day after the start of administration, the average tumor volume of tumor-bearing mice in the solvent control group reached 1746mm³, and the average tumor volume of the 5 mg/kg D-1553 administration group was 995mm³(T/C was 57.0%, TGI was 44.4%, and P value was 0.251), which showed a certain anti-tumor effect compared with the solvent control group. The average tumor volume of the 25 mg/kg IN10018 administration group was 1352mm³(T/C was 74.7%, TGI was 23.4%, and P value was 0.938), which showed no anti-tumor effect compared with the solvent control group. The average tumor volume of the 10 mg/kg INX0082 administration group was 920mm³(T/C was 54.7%, TGI was 48.9%, and P was 0.348), which also had a certain anti-tumor effect compared with the solvent control group. The average tumor volume of the combined administration group of D-1553, 5 mg/kg and IN10018, 25 mg/kg was 650mm³(T/C was 36.2%, TGI was 64.8%, and P was 0.063), which showed better anti-tumor effect compared with the solvent control group. The average tumor volume of the combined administration group of D-1553, 5 mg/kg and INX0082, 10 mg/kg was 668mm³(T/C was 39.2%, TGI was 63.8%, and P was 0.053), which also had a better anti-tumor effect compared with the solvent control group. The average tumor volume of the combined administration group of D-1553, 5 mg/kg and IN10018, 25 mg/kg, and INX0082, 10 mg/kg was 412mm³(T/C was 22.9%, TGI was 78.9%, and P was 0.016), which showed significant anti-tumor effect compared with the solvent control group, as shown in Table 7 and Figure 13.

**Table 7:**

| Group | Tumor volume (mm³) (Day 0) | Tumor volume (mm³)^{a} (Day 19) | TGI (%) (Day 19) | T/C (%) (Day 19) | p value^{b} |
|---|---|---|---|---|---|
| Solvent | 55±4 | 1746±261 | - | - | - |
| D-1553, 5 mg/kg | 55±3 | 995±143 | 44.4 | 57.0 | 0.251 |
| IN10018, 25 mg/kg | 55±3 | 1352±284 | 23.4 | 74.7 | 0.938 |
| D-1553, 5 mg/kg + IN10018, 25 mg/kg | 55±4 | 650±187 | 64.8 | 36.2 | 0.063 |
| INX0082, 10 mg/kg | 55±4 | 920±264 | 48.9 | 54.7 | 0.348 |
| D-1553, 5 mg/kg + INX0082, 10 mg/kg | 55±4 | 668±139 | 63.8 | 39.2 | 0.053 |
| D-1553, 5 mg/kg + IN10018, 25 mg/kg + INX0082, 10 mg/kg | 55±4 | 412±109 | 78.9 | 22.9 | 0.016 |

| | | | | | |
|---|---|---|---|---|---|
| Note: a. Average±SEM, n=7. | | | | | |

b. The p value (one-way ANOVA) was calculated according to the tumor volume.

### Example 9: Study of in vivo anti-tumor efficacy of combined therapy among assay substances MRTX1133, anti-mouse TIGIT antibody and IN10018 in BALB/c mouse subcutaneous allograft tumor model of mouse colon cancer CT26 cells.

### Assay materials:

The information of assay animals was shown in Table 8.

**Table 8:**

| | |
|---|---|
| Strain | BALB/c mice |
| Weight/age | 6-9 weeks old |
| Sex | female |
| Amount | 85 (including the remaining mice after grouping) |
| Supplier | Beijing Vital River Laboratory Animal Technology Co., Ltd. |

The feeding environment was shown in Table 9.

**Table 9:**

| | |
|---|---|
| Animal quarantine | After arrival, the animals were adapted to the assay environment for at least 3 days before starting the assay. |
| Temperature | 20-26°C |
| Humidity | 40-70% |
| Illumination period | 12 hours of light, 12 hours of no light |
| Cage information | The cage was made of polycarbonate with a volume of 300 mm× 180 mm× 150 mm. The padding was corncob, which was replaced twice a week. All cages and padding should be sterilized before use. |
| Cage sign | Animal information card for each cage indicated the number, sex, strain, receiving date, administration plan, assay number, group and assay start date of the animals in the cage. |
| Food and drinking water | Assay animals can freely eat (radiation sterilization, dry granular food) and drink sterilized water during the whole assay. |
| Feeding density | 5 mice/cage |
| Animal identification | Assay animals were identified by ear tags. |

The information of the assay substance was shown in Table 10.

**Table 10:**

| Name | MRTX1133 | IN10018 | Anti-mouse TIGIT antibody |
|---|---|---|---|
| Code | MRTX | IN10018 | TIGIT, TIGIT antibody |
| Supplier | SuperLan Chemical Co., Ltd. | Synthesized according to the method in patent WO2010058032 | BIOINTRON |

| Lot number/article number | Lot: 131005 | Lot: CR-C18091729-B19001 | Lot: 20220501X305 |
|---|---|---|---|
| Character | White powder | White powder | Clear transparent liquid |
| Concentration | | | 9.48 mg/ml |
| Solvent | 10% DMSO+5% Tween 80+40% PEG300 +45% PBS | DPBS | DPBS |
| Storage conditions | RT | RT | -80°C |
| Amount of usage this time | 56mg | 210mg | 10mg |

Information of the related accessories was shown in Table 11.

**Table 11:**

| Name | Code | Source | Storage condition |
|---|---|---|---|
| Polyethylene glycol 300 | PEG300 | Sinopharm (30150728) | RT |
| Dimethyl sulfoxide | DMSO | SIGMA (D2660) | RT |
| Tween 80 | Tween-80 | Shanghai Aladdin Biochemical Technology Co.,Ltd. (T104866) | RT |
| Dulbecco's phosphate buffer solution | DPBS | Shanghai Basalmedia Technologies Co.,Ltd. (B210KJ) | 4°C |

### Assay methods and steps:

Mouse colorectal cancer cell CT26 (from Nanjing Kebai Biotechnology Co., Ltd., article number: CBP60043) was maintained and passaged by InxMed (Nanjing) Co., Ltd. Cells were cultured in monolayer in vitro, and the culture conditions were RPMI-1640 medium with 10% fetal bovine serum and 37 °C 5% CO₂ incubator. Trypsin-EDTA was used for routine digestion and passage two or three times a week. When the cells were in exponential growth period and the saturation reached 80%-90%, the cells were collected, counted and inoculated.

0.1 mL of cell suspension containing 3×10⁵ cells was subcutaneously inoculated on the right back of each mouse. When the tumor volume reached about ~57 mm³ (13th day after cell inoculation), mice were randomly divided into groups according to the tumor volume and administered. The grouping information was shown in Table 12.

**Table 12:**

| Gro up | N¹ | Drug to be assayed | Dose (mg/kg) | Dosage volume parameter (mL/kg)² | Route of administ ration | Frequency of administration |
|---|---|---|---|---|---|---|
| 1 | 6 | Control | N/A | 10 | IP | QD x 14 days |
| 2 | 6 | MRTX1133 | 5 | 10 | IP | QD x 16 days |
| 3 | 6 | TIGIT | 3 | 10 | IP | BIW x 16 days |
| 4 | 6 | MRTX1133+IN10018 | 5+25 | 10 | IP+ PO | QD x 16 days +QD x 16 days |
| 5 | 6 | MRTX1133+TIGIT | 5+3 | 10 | IP+IP | QD x 16 days +BIW x 16 days |
| 6 | 6 | IN10018+TIGIT | 25+3 | 10 | PO+IP | QD x 16 days +BIW x 16 days |
| 7 | 6 | MRTX1133+IN10018 +TIGIT | 5+25+3 | 10 | IP+ PO+IP | QD x 16 days +QD x 16 days +BIW x 16 days |

| | | | | | | |
|---|---|---|---|---|---|---|
| Note: 1. N: the number of mice in each group; 2. Dosage volume: 10 mL/kg according to the body weight of mice. If the body weight loss exceeded 15%, the administration should be stopped; When the body weight returned to the initial value, the administration will be resumed. | | | | | | |

The formulation information of the assay substance and control solvent was shown in Table 13.

**Table 13:**

| Name | Dose (mg/kg) | Concen tration (mg/ml) | Formulation Method | Storage Conditions and Formulation Frequency |
|---|---|---|---|---|
| Control group | N/A | N/A | 2.4 ml of DMSO, 9.6 ml of PEG300, 1.2 ml of Tween-80 and 10.8 ml of PBS were added in sequence, and the mixture was vortexed and mixed well. | Stored at 4°C, and prepared once every 4 days. |
| MRTX1133 | 5 | 0.5 | 12.0 mg of MRTX1133 powder was weighed and 2.4 ml of DMSO was added. The mixture was mixed well with ultrasound. 9.6 ml of PEG-300, 1.2 ml of Tween-80 and 10.8 ml of DPBS were added in sequence. The mixture was mixed well by blowing to obtain a clear solution. | Stored at 4°C, and prepared once every 4 days. |
| IN10018 | 25 | 2.5 | 45 mg of IN10018 was weighed and 18 mL of DPBS was added for dilution. The mixture was mixed well to obtain a clear solution. | Stored at 4°C, and prepared once every 3~4 days. |
| TIGIT | 3 | 0.3 | 0.19 mL of the TIGIT antibody (9.48 mg/mL) was weighed and 5.81 mL of DPBS was added for dilution. The mixture was mixed well to obtain a clear solution. | Freshly prepared when used. |

### Daily observation of assay animals:

The health status and death of animals was monitored every day. Routine examination included observing the effects of tumor growth and drug treatment on the daily behavior of animals, such as behavioral activities, food and water intake (visual observation only), body weight change, appearance signs or other abnormal conditions. Based on the number of animals in each group, the death number and side effects of animals in the group were recorded.

### Assay terminating:

If the animal's health continues to deteriorate, or the tumor volume exceeds 3,000 mm³, or the animal has a serious disease or pain, the animal must be euthanized. The veterinarian should be notified and the animal should be euthanized if the following situations occur: obvious emaciation, weight loss greater than 20%; unable to eat and drink freely; the average tumor volume in the control group reaches 3,000 mm³, and the assay is terminated; the animal exhibits the following clinical manifestations and continues to worsen: standing hair, hunching back, the color of ears, nose, eyes or feet turns to white, shortness of breath, convulsions, continuous diarrhea, dehydration, slow movement and vocalization.

### Tumor measurement and assay index:

The assay index was to investigate whether the tumor growth is inhibited, delayed or cured. The diameter of tumor was measured with vernier caliper, 2~3 times a week. The formula for calculating the tumor volume is: V = 0.5 × a × b², where a and b represent the long and short diameters of the tumor respectively.

The anti-tumor effect of the compound was evaluated by TGI (%), which reflected the tumor growth inhibition rate. According to the tumor volume on the first day after grouping, the tumor growth inhibition rate TGI (%) was calculated according to the following formula: TGI (%)=[1-(the average tumor volume of an administration group-the average tumor volume of the administration group at the beginning of treatment)/(the average tumor volume of a solvent control group-the average tumor volume of the solvent control group at the beginning of treatment)]×100%.

### Statistical analysis:

Statistical analysis was based on the tumor volume at the end of the assay and analyzed by Prism Graphpad software. The comparison between multiple groups was analyzed by Two-way ANOVA and Fisher's LSD test. P < 0.05 is considered to have significant difference.

### Assay results:

The study on the in vivo efficacy of combined therapy between MRTX1133 and IN10018 in the BALB/c mouse subcutaneous allograft tumor model of mouse colorectal cancer CT-26.

After cell inoculation, the tumor growth was observed every day, and the animals were divided into groups according to the tumor volume on the 13th day after inoculation. The average tumor volume of the enrolled mice was about 57 mm³. Due to the tumor load, the animals in the control group were euthanized on the 27th day after inoculation, that is, the 14th day after group administration. The rest animals in the treatment groups were euthanized on the 29th day after inoculation, that is, the 16th day after group administration, and thus the whole assay ended.

On the 14th day after group administration, the tumor volume of the control group was 2799.4±687.0 mm³. The tumor volume of MRTX1133 (5 mg/kg) single drug group was 1067.8±302.1 mm³; the tumor volume of MRTX1133+IN10018 (5+25 mg/kg) combined group was 674.9±189.0 mm³. Comparing the comprehensive tumor volume on day 14 with the control group, the tumor inhibition rate TGI of MRTX1133 (5 mg/kg) single drug group was 63.2% (P < 0.0001); the tumor inhibition rate TGI of MRTX1133+IN10018 (5+25 mg/kg) combination group was 77.5% (p < 0.0001), both of which had significant statistical differences, as shown in Figure 14.

On the 14th day after group administration, the animals in the control group were euthanized because the tumor was too large, and the animals in the other administration groups continued to be administered until the 16th day. The tumor volume of MRTX1133 (5 mg/kg) single drug group was 1672.1±548.1 mm³; the tumor volume of MRTX1133+IN10018 (5+25 mg/kg) combined group was 1013.5±351.8 mm³. Statistical comparison between the two groups showed that the P value was p < 0.0001, indicating significant statistical difference, as shown in Figure 14.

The evaluation of tumor inhibition effect of each group was shown in Table 14. The tumor volumes of each dose group at different time periods were shown in Figure 14. Based on the whole administration period, the tumor volume of the combined treatment group of MRTX1133+IN10018 (5+25 mg/kg) was smaller than that of the single drug treatment group of MRTX1133 (5 mg/kg), and there were statistical differences. It shows that the combined administration of MRTX1133+IN10018 (5+25 mg/kg) has a better inhibitory effect on tumor growth, and 25 mg/kg of IN10018 can obviously enhance the combined treatment of MRTX1133+IN10018 (5+25 mg/kg).

**Table 14: Evaluation of the anti-tumor effect of the assay substance on BALB/c mouse transplanted tumor model of mouse colon cancer CT26 cells (based on the data on the 14th/16th day after group administration)**

| Group | Tumor volume on Day 0 (mm³)¹ | Tumor volume on Day 14 (mm³)¹ | TGI (%)² | P Value³ | Tumor volume on Day 16 (mm³)¹ | P Value ⁴ |
|---|---|---|---|---|---|---|
| Control group | 57.1±12.1 | 2799.4 ± 687.0 | / | / | / | / |
| MRTX1133 | 57.4±10.8 | 1067.8 ± 302.1 | 63.2 | <0.0001 **** | 1672.1 ± 548.1 | <0.0001 **** |
| MRTX1133+IN 10018 | 56.7±10.0 | 674.9 ± 189.0 | 77.5 | <0.0001 **** | 1013.5±351.8 | / |

| | | | | | | |
|---|---|---|---|---|---|---|
| Note: 1. The results were calculated according to the days after group administration, and the data were mean±standard deviation (mean±SD); 2. TGI (%) = [1-(T₁₄-T₀)/(V₁₄-V₀)×100%; 3. ****: P < 0.0001, vs. Control group, Two-way ANOVA; 4. ****: P < 0.0001, vs. MRTX1133+IN10018 (5+25 mg/kg) group, Two-way ANOVA; | | | | | | |

Study on the in vivo efficacy of the combined therapy of MRTX1133, TIGIT and IN10018 in BALB/c mouse subcutaneous allograft tumor model of mouse colorectal cancer CT26.

After cell inoculation, the tumor growth was observed every day. As above, on the 13th day after inoculation, the animals were grouped according to the tumor volume, and the average tumor volume of enrolled mice was about 57 mm³. Due to the tumor load, the animals in the control group were euthanized on the 27th day after inoculation, that is, the 14th day after group administration. The rest animals in the other treatment groups were euthanized on the 29th day after inoculation, that is, the 16th day after group administration, and thus the whole assay ended.

On the 14th day after group administration, the tumor volume of the control group was 2799.4±687.0mm³. The tumor volumes of MRTX1133 (5 mg/kg) and TIGIT (3 mg/kg) single drug groups were 1067.8±302.1 mm³ and 976.8±640.4 mm³ respectively. The tumor volumes of MRTX1133+IN10018 (5+25 mg/kg) and IN10018+TIGIT (25+3 mg/kg) combined treatment groups were 674.9±189.0mm³ and 1315.6±870.2mm³, respectively. The tumor volume of MRTX1133+IN10018+TIGIT (5+25+3 mg/kg) combined group was 535.5±288.9 mm³. Comparing the comprehensive tumor volume with the control group, the tumor inhibition rate TGI of MRTX1133 (5 mg/kg) and TIGIT (3 mg/kg) in single drug groups were 63.2% (p < 0.0001) and 66.5% (P < 0.0001) respectively. The tumor inhibition rate TGI of MRTX1133+IN10018 (5+25 mg/kg) and IN10018+TIGIT (25+3 mg/kg) in the combination group of two drugs were 77.5% (p < 0.0001) and 54.1% (P < 0.0001) respectively. In the three-drug combination group of MRTX1133+IN10018+TIGIT (5+25+3 mg/kg), the tumor inhibition rate TGI was 82.5% (p < 0.0001). All results were statistically significant.

On the 14th day after group administration, the animals in the control group were euthanized because the tumor was too large, and the animals in the other groups continued to be administered until the 16th day. The tumor volumes of MRTX1133 (5 mg/kg) and TIGIT (3 mg/kg) in single drug groups were 1672.1±548.1 mm³ and 1225.8±814.5 mm³ respectively. The tumor volumes of MRTX1133+IN10018 (5+25 mg/kg) and IN10018+TIGIT (25+3 mg/kg) in two-drug combined groups were 1013.5±351.8mm³ and 1857±1240.4mm³, respectively. The tumor volume of MRTX1133+IN10018+TIGIT (5+25+3 mg/kg) in three-drug combined group was 809.3±511.4 mm³. Compared with the combined group of MRTX1133+IN10018+TIGIT (5+25+3mg/kg), the comprehensive tumor volume was statistically analyzed. The P values of MRTX1133 (5 mg/kg) and TIGIT (3 mg/kg) in single drug groups were p < 0.0001 and p =0.0339, respectively. The p values of MRTX1133+IN10018 (5+25 mg/kg) and IN10018+TIGIT (25+3 mg/kg) in two-drug combined groups were p =0.2965 and p < 0.0001, respectively. The results have statistical differences compared with the combined treatment group except the MRTX1133+IN10018 (5+25 mg/kg) group.

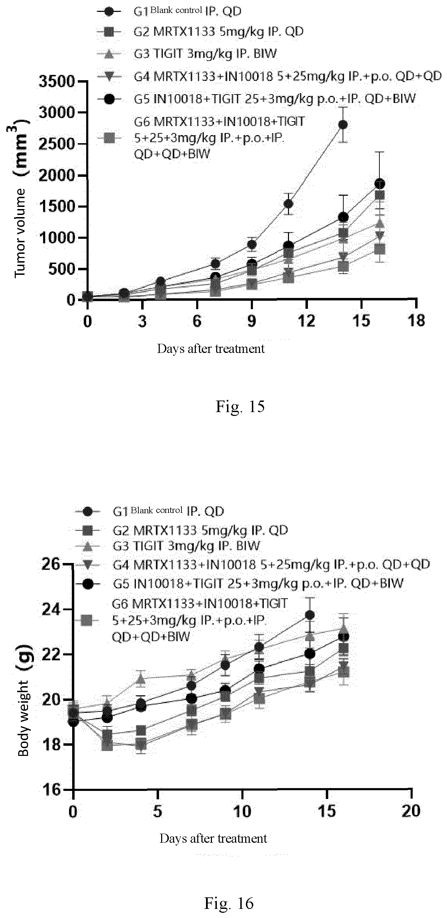

The evaluation of tumor inhibition effect of each group was shown in Table 15. The tumor volume of each dose group at different time periods was shown in Figure 15. Considering the whole administration period, the tumor volume of MRTX1133+IN10018+TIGIT (5+25+3 mg/kg) combined treatment group was also one of the relatively smallest groups.

**Table 15: Evaluation of the anti-tumor effect of the assay substance on BALB/c mouse transplanted tumor model of mouse colon cancer CT26 cells (based on the data on the 14th/16th day after group administration)**

| Group | Tumor volume on day 0 (mm³)¹ | Tumor volume on day 14 (mm³)¹ | TGI (%)² | P Value³ | Tumor volume on day 16 (mm³)¹ | P Value⁴ |
|---|---|---|---|---|---|---|
| Control group | 57.1±12.1 | 2799.4 ± 687.0 | / | / | / | / |
| MRTX1133 | 57.4±10.8 | 1067.8 ± 302.1 | 63.2 | <0.0001 **** | 1672.1 ± 548.1 | <0.0001* *** |
| TIGIT | 56.7±11.6 | 976.8±640.4 | 66.5 | <0.0001 **** | 1225.8±814.5 | 0.0339* |
| MRTX1133+IN10 018 | 56.7±10.0 | 674.9 ± 189.0 | 77.5 | <0.0001 **** | 1013.5±351.8 | 0.2965 |
| IN10018+TIGIT | 55.7±11.7 | 1315.6 ± 870.2 | 54.1 | <0.0001 **** | 1857±1240.4 | <0.0001* *** |
| MRTX1133+IN10 018+TIGIT | 55.7±10.9 | 535.5 ± 288.9 | 82.5 | <0.0001 **** | 809.3±511.4 | / |

| | | | | | | |
|---|---|---|---|---|---|---|
| Note: 1. The results were calculated according to the days after group administration, and the data were mean±standard deviation (mean±SD); 2. TGI (%) = [1-(T₁₄-T₀)/(V₁₄-V₀)] ×100%; 3. ****: P < 0.0001, vs. Control group, Two-way ANOVA; 4. *: p<0.05, ****: p<0.0001, vs. MRTX1133+IN10018+TIGIT (5+25+3 mg/kg) group, Two-way ANOVA; | | | | | | |

Study on the weight change and clinical status of the combined treatment among MRTX1133, TIGIT and IN10018 in the BALB/c mouse subcutaneous allograft tumor model of mouse colorectal cancer CT26.

The assay was carried out according to the administration scheme. During the assay, animal activities such as eating and drinking were observed daily, and animal weight was recorded 2~3 times a week. On the day of euthanasia, that is, the 14th day after group administration in the control group, and the 16th day after group administration in the other administration groups, the body weight of the control group changed from 19.4g on the day of group administration (Day 0) to 23.7g on the 14th day, with a weight change rate of 22.0%; the average body weight of MRTX1133 (5 mg/kg) and TIGIT (3 mg/kg) single drug groups changed from 19.4g and 19.6g on the day of group administration (Day 0) to 22.3g and 23.1g on the 16th day, and the change rates of body weight were 15.1% and 18.2% respectively. The body weight of the two-drug combined groups of MRTX1133+IN10018 (5+25 mg/kg) and IN10018+TIGIT (25+3 mg/kg) changed from 19.4g and 19.0g on Day 0 to 21.4g and 22.8g on 16th day, respectively, and the change rates of body weight were 10.7% and 19.6%, respectively. The body weight of the three-drug combined group of MRTX1133+IN10018+TIGIT (5+25+3 mg/kg) group changed from 19.5g on the day of group administration (Day 0) to 21.2g on the 16th day, and the change rate of body weight was 8.5%.

During the whole administration period, the body weight of animals in each group did not decrease significantly, the mental state of animals was good, and the feeding and movement were normal. The body weight data of each group were shown in Table 16, and the change and change rate of the body weight of each dose group in different time periods were shown in Figure 16. The body weight of animals in each group did not decrease significantly, the mental state of animals was good, and the feeding and movement were normal, showing tolerance to the combined treatment between MRTX1133 (5 mg/kg), TIGIT (3 mg/kg) and IN10018 (25 mg/kg).

**Table 16: Evaluation of the effect of the assay substance on body weight change in BALB/c mouse transplanted tumor model of mouse colon cancer CT26 cells (based on the data on the 14th/16th day after group administration)**

| Group | Animal survival¹ | Weight on Day 0(g)² | Weight on Day 14/16 (g)³ | Weight change rate (%)⁴ |
|---|---|---|---|---|
| Control group | 6/6 | 19.4±0.8 | 23.7±1.9 | 22.0 |
| MRTX1133 | 6/6 | 19.4±0.8 | 22.3±0.8 | 15.1 |
| TIGIT | 6/6 | 19.6±0.9 | 23.1±1.6 | 18.2 |
| MRTX1133+IN10018 | 6/6 | 19.4±0.5 | 21.4±0.9 | 10.7 |
| IN10018+TIGIT | 6/6 | 19±0.3 | 22.8±2.0 | 19.6 |
| MRTX1133+IN10018+ TIGIT | 6/6 | 19.5±0.8 | 21.2±1.5 | 8.5 |

| | | | | |
|---|---|---|---|---|
| Note: 1. The results were calculated according to the days after group administration, the number of animals alive on day 0/the number of animals alive on day 14 (control group) or day 16 (other administration groups); 2. The data were mean±standard deviation (mean±SD); 3. The control group was calculated based on the data of the 14th day after group administration, and the other administration groups were calculated based on the data of the 16th day after group administration; 4. Weight change rate = [1-(W_{14/16}-W₀) / W₀]*100%; | | | | |

### Example 10: Study on the Induction of Immunogenic Cell Death Targets of Mouse Breast Cancer 4T1 Cells in Vitro by Assay Compounds Lurbinectedin and AMP945.

### Assay materials:

### 1) Drugs used in this assay

Lurbinectedin was provided by DC Chemicals, Lot No.: DC12502.

AMP945 was provided by MCE, Lot No.: 143253.

### 2) Antibodies used in this assay

Recombinant Alexa Fluor^{®} 647 fluorescent Anti-Calreticulin antibody (Abcam, Cat No.: ab196159, Lot No.: CR33676773). Annexin V-apoptosis detection kit (Beyotime, Cat No.: C1062L, Lot No.: 122221220706).

### Assay method:

4T1 cells (from Nanjing Kebai Biotechnology Co., Ltd., article number: CBP60352) were cultured with RPMI 1640 (Shanghai Basalmedia, Cat No.: L210KJ, Lot No.: F210916)+10%FBS (Gibco, Cat No.: 10099-141c, Lot No.: 2158737cp), wherein the culture conditions were 37 °C and 5% CO₂. Trypsin was used for routine digestion and passage treatment two or three times a week. When the cells were in exponential growth period and the adherent confluence reached 80%-90%, the cells were collected and plated. 4T1 cells were digested with trypsin. The cells were then collected and counted. According to the counting results, the cells were diluted with RPMI-1640+10%FBS, and the dilution concentration was 50,000 cells per ml. Then, the cells were plated on 12-well culture plates, and 2 ml of cell suspension (100,000 cells) was laid in each well. After plating, the cells were cultured in 37 °C and 5% CO₂ incubator. After the cells were spread for 24 hours, six groups were set up, wherein the first group was a control group, and the culture medium was added; the second group was AMP945 with a concentration of 3µM; the third group was AMP945 with a concentration of 6 µM; the fourth group was Lurbinectedin with a concentration of 0.002 µM; the fifth group was AMP945(3 µM) combined with Lurbinecedin (0.002 µM); the sixth group was AMP945(6 µM) combined with Lurbinecedin (0.002 µM). The drugs were mixed and the mixture was cultured in a 5%CO₂ incubator at 37°C for 48 hours.

### Assay result

After 48 hours of drug action, the cells were collected for flow analysis. The cells were washed twice with flow buffer (PBS+2%FBS). 0.5 µl of AF647 anti-Calreticulin antibody (abcam) was added to each well, and mixed. The cells were incubated at 4°C in the dark. After 20 min of incubation, the flow buffer was added. Annexin staining kit (Beyotime) was used, and 195 µl of Annexin-V-FITC conjugate was added. The cells were blown and mixed, and 5 µl of Annexin-V-FITC antibody was added. The cells were gently mixed. Finally, 10 µl of PI dye was added and mixed, and the cells were incubated in the dark at room temperature for 15 min. The sample was sent to a flow cytometer for signal determination.

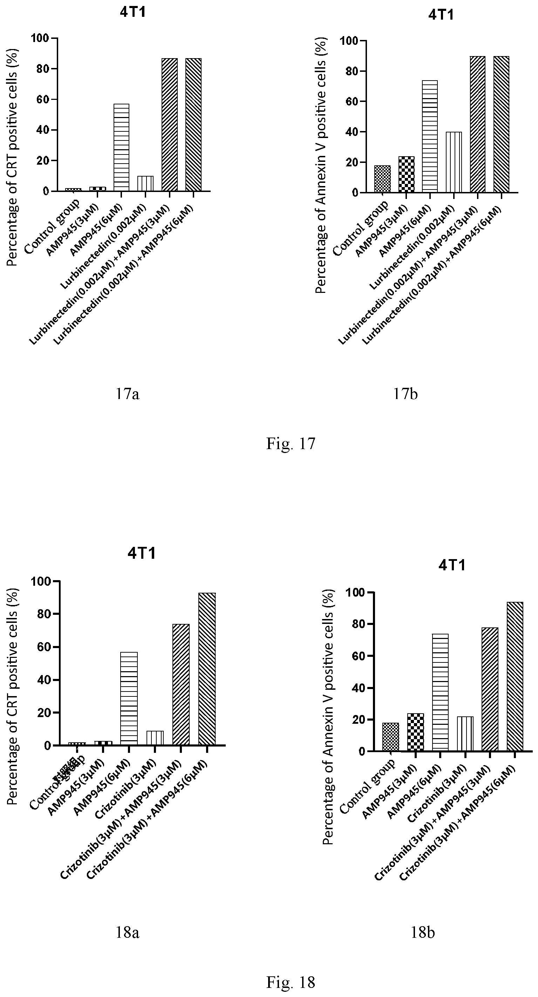

The results of flow cytometry analysis showed that the positive rates of CRT and Annexin-V in the two-drug combination group were significantly better than those in the single drug group and the control group, as shown in Figure 17.

### Example 11: Study on the induction of Immunogenic Cell Death Targets of Mouse Breast Cancer 4T1 Cells in vitro by Assay Compounds Crizotinib and AMP945.

### Assay materials:

### 1) Drugs used in this assay

Crizotinib was provided by MCE, Lot No.: 06049.

AMP945 was provided by MCE, Lot No.: 143253.

### 2) Antibodies used in this assay

Recombinant Alexa Fluor^{®} 647 fluorescent Anti-Calreticulin antibody (Abcam, Cat No.: ab196159, Lot No.: CR33676773). Annexin V-apoptosis detection kit (Beyotime, Cat No.: C1062L, Lot No.: 122221220706).

### Assay method:

4T1 cells (from Nanjing Kebai Biotechnology Co., Ltd., article number: CBP60352) were cultured with RPMI 1640 (Shanghai Basalmedia, Cat No.: L210KJ, Lot No.: F210916)+10%FBS (Gibco, Cat No.: 10099-141c, Lot No.: 2158737cp), wherein the culture conditions were 37 °C and 5% CO₂. Trypsin was used for routine digestion and passage treatment two or three times a week. When the cells were in exponential growth period and the adherent confluence reached 80%-90%, the cells were collected and plated. 4T1 cells were digested with trypsin. The cells were then collected and counted. According to the counting results, the cells were diluted with RPMI-1640+10%FBS, and the dilution concentration was 50,000 cells per ml. Then, the cells were plated on 12-well culture plates, and 2 ml of cell suspension (100,000 cells) was laid in each well. After plating, the cells were cultured in 37 °C and 5% CO₂ incubator. After the cells were spread for 24 hours, six groups were set up, wherein the first group was a control group, and the culture medium was added; the second group was AMP945 with a concentration of 3µM; the third group was AMP945 with a concentration of 6 µM; the fourth group was Crizotinib with a concentration of 3 µM; the fifth group was AMP945(3 µM) combined with Crizotinib (3 µM); the sixth group was AMP945(6 µM) combined with Crizotinib (3 µM). The drugs were mixed and the mixture was cultured in a 5%CO₂ incubator at 37°C for 48 hours.

### Assay result

After 48 hours of drug action, the cells were collected for flow analysis. The cells were washed twice with flow buffer (PBS+2%FBS). 0.5 µl of AF647 anti-Calreticulin antibody (abcam) was added to each well, and mixed. The cells were incubated at 4°C in the dark. After 20 min of incubation, the flow buffer was added. Annexin staining kit (Beyotime) was used, and 195 µl of Annexin-V-FITC conjugate was added. The cells were blown and mixed, and 5 µl of Annexin-V-FITC antibody was added. The cells were gently mixed. Finally, 10 µl of PI dye was added and mixed, and the cells were incubated in the dark at room temperature for 15 min. The sample was sent to a flow cytometer for signal determination.

The results of flow cytometry analysis showed that the positive rates of CRT and Annexin-V in the two-drug combination group were significantly better than those in the single drug group and the control group, as shown in Figure 18.

All references mentioned in the present disclosure are incorporated by reference in their entirety as if each document was listed separately. It should be understood that after reading the present disclosure, those skilled in the art can make various changes or modifications to the present disclosure, and these equivalents also fall within the scope defined by the claims of this application.

## Claims

1. Use of an FAK inhibitor, an immunogenic cell death inducer and an immune checkpoint inhibitor in the manufacture of a medicament for treating a tumor in a subject, wherein the immunogenic cell death inducer is an RNA polymerase II inhibitor, an ALK/ROS1 inhibitor, a KRAS G12C inhibitor, or a KRAS G12D inhibitor.

2. A pharmaceutical combination product of an FAK inhibitor, an immunogenic cell death inducer and an immune checkpoint inhibitor for use in treating a tumor in a subject, wherein the immunogenic cell death inducer is an RNA polymerase II inhibitor, an ALK/ROS1 inhibitor, a KRAS G12C inhibitor, or a KRAS G12D inhibitor.

3. A method for treating a tumor in a subject, comprising administering to the subject a therapeutically effective amount of an FAK inhibitor, an immunogenic cell death inducer and an immune checkpoint inhibitor, wherein the immunogenic cell death inducer is an RNA polymerase II inhibitor, an ALK/ROS1 inhibitor, a KRAS G12C inhibitor, or a KRAS G12D inhibitor.

4. The use, the pharmaceutical combination product, or the method according to any one of claims 1-3, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, and yet alternatively, the FAK inhibitor is IN10018, or a pharmaceutically acceptable salt thereof, alternatively, the FAK inhibitor is IN10018 tartrate, wherein the IN10018 has a structure of:

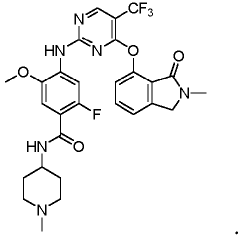

5. The use, the pharmaceutical combination product, or the method according to any one of claims 1-4, wherein the immunogenic cell death inducer is an RNA polymerase II inhibitor.

6. The use, the pharmaceutical combination product, or the method according to claim 5, wherein the RNA polymerase II inhibitor is Lurbinectedin, SEL-120, or a pharmaceutically acceptable salt thereof.

7. The use, the pharmaceutical combination product, or the method according to claim 5 or 6, wherein the RNA polymerase II inhibitor is Lurbinectedin.

8. The use, the pharmaceutical combination product, or the method according to any one of claims 1-4, wherein the immunogenic cell death inducer is an ALK/ROS1 inhibitor.

9. The use, the pharmaceutical combination product, or the method according to claim 8, wherein the ALK/ROS1 inhibitor is Crizotinib, SIM-0201, XZP-3621, TQ-B3139,SAF-189s, Ceritinib, Lorlatinib (PF-06463922), Alectinib, Ensartinib, APG-2449, Brigatinib, TQ-B3101, Entrectinib, Repotrectinib, or a pharmaceutically acceptable salt thereof, alternatively, the an ALK/ROS1 inhibitor is Crizotinib, Entrectinib, or a pharmaceutically acceptable salt thereof.

10. The use, the pharmaceutical combination product, or the method according to claim 8 or 9, wherein the ALK/ROS1 inhibitor is Crizotinib.

11. The use, the pharmaceutical combination product, or the method according to any one of claims 1-4, wherein the immunogenic cell death inducer is a KRAS G12C inhibitor.

12. The use, the pharmaceutical combination product, or the method according to claim 11, wherein the KRAS G12C inhibitor is D-1553, ARS-3248, GF-105, JAB-21822, JDQ-443, LY-3537982, Sotorasib (AMG510), Adagrasib (MRTX849), GDC-6036, or a pharmaceutically acceptable salt thereof, alternatively, the KRAS G12C inhibitor is D-1553, Sotorasib (AMG510), or a pharmaceutically acceptable salt thereof.

13. The use, the pharmaceutical combination product, or the method according to any one of claims 11-12, wherein the KRAS G12C inhibitor is D-1553, or a pharmaceutically acceptable salt thereof.

14. The use, the pharmaceutical combination product, or the method according to any one of claims 1-4, wherein the immunogenic cell death inducer is a KRAS G12D inhibitor.

15. The use, the pharmaceutical combination product, or the method according to claim 14, wherein the KRAS G12D inhibitor is MRTX1133, HRS-4642, JAB-22000, or a pharmaceutically acceptable salt thereof.

16. The use, the pharmaceutical combination product, or the method according to any one of claims 14-15, wherein the KRAS G12D inhibitor is MRTX1133, or a pharmaceutically acceptable salt thereof.

17. The use, the pharmaceutical combination product, or the method according to any one of claims 1-16, wherein the immune checkpoint inhibitor is an anti-PD-1/PD-L1 antibody, a PD-1/PD-L1 small molecule inhibitor, or a TIGIT antibody.

18. The use, the pharmaceutical combination product, or the method according to any one of claims 1-17, wherein the immune checkpoint inhibitor is an anti-PD-1/PD-L1 antibody, alternatively, the anti-PD-1/PD-L1 antibody is pembrolizumab, tislelizumab, nivolumab, toripalimab, atezolizumab, durvalumab, avelumab, Atezolizumab, camrelizumab, sintilimab, cemiplimab, envafolimab, BMS-936559, JS003, SHR-1316, GS-4224, AN-4005, or MX-10181.

19. The use, the pharmaceutical combination product, or the method according to any one of claims 1-17, wherein the immune checkpoint inhibitor is a PD-1/PD-L1 small molecule inhibitor, alternatively, the PD-1/PD-L1 small molecule inhibitor is INCB-086550, Lazertinib, IMMH-010, CA-170, ABSK043, or RRx-001.

20. The use, the pharmaceutical combination product, or the method according to any one of claims 1-17, wherein the immune checkpoint inhibitor is a TIGIT antibody, alternatively, the TIGIT antibody is Ociperlimab (BGB-A1217), Vibostolimab, domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.

21. The use, the pharmaceutical combination product, or the method according to any one of claims 1-4, wherein the FAK inhibitor is IN10018 or a pharmaceutically acceptable salt thereof, the immunogenic cell death inducer is Lurbinectedin; and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.

22. The use, the pharmaceutical combination product, or the method according to any one of claims 1-4, wherein the FAK inhibitor is IN10018 or a pharmaceutically acceptable salt thereof, the immunogenic cell death inducer is Crizotinib or a pharmaceutically acceptable salt thereof, and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.

23. The use, the pharmaceutical combination product, or the method according to any one of claims 1-4, wherein the FAK inhibitor is IN10018 or a pharmaceutically acceptable salt thereof, the immunogenic cell death inducer is D-1553 or a pharmaceutically acceptable salt thereof, and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.

24. The use, the pharmaceutical combination product, or the method according to any one of claims 1-4, wherein the FAK inhibitor is IN10018 or a pharmaceutically acceptable salt thereof, the immunogenic cell death inducer is MRTX1133 or a pharmaceutically acceptable salt thereof, and the immune checkpoint inhibitor is a TIGIT antibody.

25. The use, the pharmaceutical combination product, or the method according to any one of claims 1-24, wherein the FAK inhibitor, the immunogenic cell death inducer and the immune checkpoint inhibitor are administered simultaneously or sequentially to the subject.

26. The use, the pharmaceutical combination product, or the method according to any one of claims 1-25, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), gastric cancer, melanoma, or pancreatic cancer.

27. The use, the pharmaceutical combination product, or the method according to any one of claims 1-26, wherein the tumor is lung cancer, colon cancer (including colorectal cancer) or breast cancer.

28. A kit or pharmaceutically acceptable composition, comprising:
(a) an FAK inhibitor;
(b) an immunogenic cell death inducer; and
(c) an immune checkpoint inhibitor,
wherein the immunogenic cell death inducer is an RNA polymerase II inhibitor, an ALK/ROS1 inhibitor, a KRAS G12C inhibitor or a KRAS G12D inhibitor.

29. The kit or composition according to claim 28, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, and yet alternatively, the FAK inhibitor is IN10018, or a pharmaceutically acceptable salt thereof, alternatively, the FAK inhibitor is IN10018 tartrate, wherein the IN10018 has a structure of:

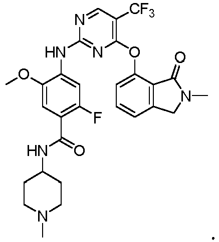

30. The kit or composition according to any one of claims 28-29, wherein the immunogenic cell death inducer is an RNA polymerase II inhibitor.

31. The kit or composition according to claim 30, wherein the RNA polymerase II inhibitor is Lurbinectedin, SEL-120, or a pharmaceutically acceptable salt thereof.

32. The kit or composition according to any one of claims 30-31, wherein the RNA polymerase II inhibitor is Lurbinectedin.

33. The kit or composition according to any one of claims 28-29, wherein the immunogenic cell death inducer is an ALK/ROS1 inhibitor.

34. The kit or composition according to claim 33, wherein the ALK/ROS1 inhibitor is Crizotinib, SIM-0201, XZP-3621, TQ-B3139, SAF-189s, Ceritinib, Lorlatinib (PF-06463922), Alectinib, Ensartinib, APG-2449, Brigatinib, TQ-B3101, Entrectinib, Repotrectinib, or a pharmaceutically acceptable salt thereof, alternatively, the ALK/ROS1 inhibitor is Crizotinib, Entrectinib, or a pharmaceutically acceptable salt thereof.

35. The kit or composition according to any one of claims 33-34, wherein the ALK/ROS1 inhibitor is Crizotinib, or a pharmaceutically acceptable salt thereof.

36. The kit or composition according to any one of claims 28-29, wherein the immunogenic cell death inducer is a KRAS G12C inhibitor.

37. The kit or composition according to claim 36, wherein the KRAS G12C inhibitor is D-1553, ARS-3248, GF-105, JAB-21822, JDQ-443, LY-3537982, Sotorasib (AMG510), Adagrasib (MRTX849), GDC-6036, or a pharmaceutically acceptable salt thereof, alternatively, the KRAS G12C inhibitor is D-1553, Sotorasib (AMG510), or a pharmaceutically acceptable salt thereof.

38. The kit or composition according to any one of claims 36-37, wherein the KRAS G12C inhibitor is D-1553, or a pharmaceutically acceptable salt thereof.

39. The kit or composition according to any one of claims 28-29, wherein the immunogenic cell death inducer is a KRAS G12D inhibitor.

40. The kit or composition according to claim 39, wherein the KRAS G12D inhibitor is MRTX1133, HRS-4642, JAB-22000, or a pharmaceutically acceptable salt thereof.

41. The kit or composition according to any one of claims 39-40, wherein the KRAS G12D inhibitor is MRTX1133, or a pharmaceutically acceptable salt thereof.

42. The kit or composition according to any one of claims 28-41, wherein the immune checkpoint inhibitor is an anti-PD-1/PD-L1 antibody, a PD-1/PD-L1 small molecule inhibitor, or a TIGIT antibody.

43. The kit or composition according to any one of claims 28-42, wherein the immune checkpoint inhibitor is an anti-PD-1/PD-L1 antibody, alternatively, the anti-PD-1/PD-L1 antibody is pembrolizumab, tislelizumab, nivolumab, toripalimab, atezolizumab, durvalumab, avelumab, atezolizumab, camrelizumab, sintilimab, cemiplimab, envafolimab, BMS-936559, JS003, SHR-1316, GS-4224, AN-4005, or MX-10181.

44. The kit or composition according to any one of claims 28-42, wherein the immune checkpoint inhibitor is a PD-1/PD-L1 small molecule inhibitor, alternatively, the PD-1/PD-L1 small molecule inhibitor is INCB-086550, Lazertinib, IMMH-010, CA-170, ABSK043, or RRx-001.

45. The kit or composition according to any one of claims 28-42, wherein the immune checkpoint inhibitor is a TIGIT antibody, alternatively, the TIGIT antibody is ociperlimab (BGB-A1217), vibostolimab, domvanalimab (AB154), tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.

46. The kit or composition according to any one of claims 28-29, wherein the FAK inhibitor is IN10018 or a pharmaceutically acceptable salt thereof, the immunogenic cell death inducer is Lurbinectedin; and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.

47. The kit or composition according to any one of claims 28-29, wherein the FAK inhibitor is IN10018 or a pharmaceutically acceptable salt thereof, the immunogenic cell death inducer is Crizotinib or a pharmaceutically acceptable salt thereof, and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.

48. The kit or composition according to any one of claims 28-29, wherein the FAK inhibitor is IN10018 or a pharmaceutically acceptable salt thereof, the immunogenic cell death inducer is D-1553 or a pharmaceutically acceptable salt thereof, and the immune checkpoint inhibitor is an anti-PD-1/PD-L1 antibody, or a PD-1/PD-L1 small molecule inhibitor, alternatively, the immune checkpoint inhibitor is an anti-PD-1/PD-L1 antibody.

49. The kit or composition according to any one of claims 28-29, wherein the FAK inhibitor is IN10018 or a pharmaceutically acceptable salt thereof, the immunogenic cell death inducer is MRTX1133 or a pharmaceutically acceptable salt thereof, and the immune checkpoint inhibitor is a TIGIT antibody.

50. The kit or composition according to any one of claims 28-49, wherein the kit or composition is used as a medicament.

51. The kit or composition according to claim 50, wherein the medicament is used for treating tumors, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), gastric cancer, melanoma, or pancreatic cancer.

52. The kit or composition according to claim 51, wherein the tumor is lung cancer, colon cancer (including colorectal cancer) or breast cancer.

53. An FAK inhibitor for use in the treatment of a tumor to enhance immunogenic cell death induced by an immunogenic cell death inducer, wherein the immunogenic cell death inducer is an RNA polymerase II inhibitor, an ALK/ROS1 inhibitor, a KRAS G12C inhibitor, or a KRAS G12D inhibitor.

54. The FAK inhibitor according to claim 53, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, alternatively, the FAK inhibitor is IN10018, or a pharmaceutically acceptable salt thereof, alternatively, the FAK inhibitor is IN10018 tartrate, wherein the IN10018 has a structure of:

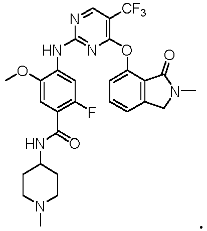

55. The FAK inhibitor according to claim 53 or 54, wherein the immunogenic cell death inducer is an RNA polymerase II inhibitor.

56. The FAK inhibitor according to claim 55, wherein the RNA polymerase II inhibitor is Lurbinectedin, SEL-120, or a pharmaceutically acceptable salt thereof, alternatively the RNA polymerase II inhibitor is Lurbinectedin.

57. The FAK inhibitor according to claim 53 or 54, wherein the immunogenic cell death inducer is an ALK/ROS1 inhibitor.

58. The FAK inhibitor according to claim 57, wherein the ALK/ROS1 inhibitor is Crizotinib, SIM-0201, XZP-3621, TQ-B3139, SAF-189s, Ceritinib, Lorlatinib (PF-06463922), Alectinib, Ensartinib, APG-2449, Brigatinib, TQ-B3101, Entrectinib, Repotrectinib, or a pharmaceutically acceptable salt thereof, alternatively, the ALK/ROS1 inhibitor is Crizotinib, Entrectinib, or a pharmaceutically acceptable salt thereof.

59. The FAK inhibitor according to any one of claims 57-58, wherein the ALK/ROS1 inhibitor is Crizotinib.

60. The FAK inhibitor according to claim 53 or 54, wherein the immunogenic cell death inducer is a KRAS G12C inhibitor.

61. The FAK inhibitor according to claim 60, wherein the KRAS G12C inhibitor is D-1553, ARS-3248, GF-105, JAB-21822, JDQ-443, LY-3537982, Sotorasib (AMG510), Adagrasib (MRTX849), GDC-6036, or a pharmaceutically acceptable salt thereof, alternatively, the KRAS G12C inhibitor is D-1553, Sotorasib (AMG510), or a pharmaceutically acceptable salt thereof.

62. The FAK inhibitor according to any one of claims 60-61, wherein the KRAS G12C inhibitor is D-1553, or a pharmaceutically acceptable salt thereof.

63. The FAK inhibitor according to claim 53 or 54, wherein the immunogenic cell death inducer is a KRAS G12D inhibitor.

64. The FAK inhibitor according to claim 63, wherein the KRAS G12D inhibitor is MRTX1133, HRS-4642, JAB-22000, or a pharmaceutically acceptable salt thereof.

65. The FAK inhibitor according to any one of claims 63-64, wherein the KRAS G12D inhibitor is MRTX1133, or a pharmaceutically acceptable salt thereof.

66. The FAK inhibitor according to any one of claims 53-65, wherein the medicament is used for treating tumors, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), gastric cancer, melanoma, or pancreatic cancer.

67. The FAK inhibitor according to claim 66, wherein the tumor is lung cancer, colon cancer (including colorectal cancer) or breast cancer.
